# EUROPEAN PATENT SPECIFICATION

(11) **EP 1 518 505 B1**
(45) Date of publication and mention of the grant of the patent: **10.12.2008**
(21) Application number: 04013897.6
(22) Date of filing: 14.06.2004
(51) Int. Cl.: A61B 17/32

(54) **Ultrasonic surgical system, and abnormality detection method and abnormality detection computer program product for ultrasonic surgical system**
Chirurgisches Ultraschallsystem und Methode zur Erkennung einer abnormalen Gerätesituation und zugehöriges Computerprogrammprodukt
Système chirurgical ultrasonique, méthode de détection d'anomalie et support avec programme d'ordinateur de detection d'anomalie pour un système chirurgical ultrasonique

(30) Priority: 29.09.2003 JP 2003337556
(43) Date of publication of application: 30.03.2005
(73) Proprietor: Olympus Corporation, Tokyo 151-0072 (JP)
(72) Inventor: Takahashi, Hiroyuki, Hachioji-shi Tokyo 192-8512 (JP)
(74) Representative: von Hellfeld, Axel

(56) References cited:
- US-A- 5 400 267
- US-A- 6 017 354
- US-A1- 2002 049 427
- US-B1- 6 308 089
- PATENT ABSTRACTS OF JAPAN vol. 2000, no. 09, 13 October 2000 (2000-10-13) & JP 2000 175926 A (TOSHIBA CORP), 27 June 2000 (2000-06-27)

## Description

### BACKGROUND OF THE INVENTION

### 1) Field of the Invention

The present invention relates to an ultrasonic surgical system which performs a surgical, medical treatment such as coagulation and incision of a biological tissue, lithotrity, and aspiration by applying an ultrasonic vibration, and an abnormality detection method and an abnormality detection program for the ultrasonic surgical system.

### 2) Description of the Related Art

There has been conventionally developed an ultrasonic surgical system which includes a handpiece having an ultrasonic vibrator incorporated therein and a probe connected to the ultrasonic vibrator, which contacts a probe, to which an ultrasonic vibration is transmitted, with a biological tissue or the like, and which conducts a surgical, medical treatment such as incision, excision, or aspiration to the biological tissue. The ultrasonic vibration output from the ultrasonic vibrator of this ultrasonic surgical system is realized by controlling this ultrasonic vibrator to be driven. Generally, it is desirable that the ultrasonic vibrator is driven at a resonance frequency or a frequency near the resonance frequency (hereinafter, "near-resonance frequency").

If an overload is imposed on a vibration system in the ultrasonic surgical system due to one of various abnormalities such as a damage of the probe, adhesion of a blood or the like, and a malfunction and the like of the handpiece, the ultrasonic vibrator of this ultrasonic surgical system is difficult to drive at the resonance frequency or the near-resonance frequency. This results in deterioration of a function of the ultrasonic surgical system to ensure performing the medical treatment. If so, the ultrasonic surgical system needs to stop driving the ultrasonic vibrator at an early timing or to alarm an operator so as to prevent recurrence or deterioration of the abnormalities.

United States Patent No. 6,017,354 describes an integrated surgical tool system having a handpiece and a surgical tool or implement attached thereto. The handpiece includes a memory which stores data regarding the operating parameters of the handpiece.

### SUMMARY OF THE INVENTION

It is an object of the present invention to at least solve the problems in the conventional technology. The present invention provides an ultrasonic surgical system as defined in claim 1, an ultrasonic surgical method as defined in claim 9, and a computer program product as defined in claim 12. Preferred features of the invention are recited in the dependent claims.

An ultrasonic surgical system according to one aspect of the present invention includes a handpiece including an ultrasonic vibrator, and a first storage unit that stores first determination criterion information being a criterion of whether an abnormality occurs in the ultrasonic surgical system; a probe including a second storage unit that stores second determination criterion information as a criterion of whether an abnormality occurs in the ultrasonic surgical system, connected to the ultrasonic vibrator, and transmitting ultrasonic vibrations output from the ultrasonic vibrator to a treatment target; and a control unit determining whether the abnormality occurs in the ultrasonic surgical system based on the first determination criterion information and the second determination criterion information, stopping driving the ultrasonic vibrator if determining that the abnormality occurs in the ultrasonic surgical system.

A method of detecting an abnormality of an ultrasonic surgical system which includes (1) a handpiece having an ultrasonic vibrator and (2) a probe connected to the ultrasonic vibrator, according to another aspect of the present invention includes determining whether an abnormality occurs in the ultrasonic surgical system based on first determination criterion information on the handpiece and second determination criterion information on the probe before the ultrasonic vibrator is driven; determining whether a resonance point of the ultrasonic vibrator is detected within a frequency range set by one of the first determination criterion information and the second determination criterion information if the abnormality is not determined before the ultrasonic vibrator is driven; determining that an abnormality occurs in the ultrasonic surgical system if the resonance point is not detected within the frequency range; determining whether an abnormality occurs to the ultrasonic surgical system based on driving information obtained during driving of the ultrasonic vibrator and based on one of the first determination criterion information and the second determination criterion information if the abnormality is not determined based on the resonance point; and stopping driving the ultrasonic vibrator when the abnormality is determined.

The computer program product according to still another aspect of the present invention realizes the method according to the present invention on a computer.

The other objects, features, and advantages of the present invention are specifically set forth in or will become apparent from the following detailed description of the invention when read in conjunction with the accompanying drawings.

### BRIEF DESCRIPTION OF THE DRAWINGS

Fig. 1 is a typical view of schematic configuration of an ultrasonic surgical system according to a first embodiment of the present invention;
Fig. 2 is a typical view of a connection state among a handpiece, a probe, and a sheath;
Fig. 3 is a longitudinal sectional, typical view of an arrangement state of a storage unit arranged in the probe;
Fig. 4 is a block diagram of basic configuration of the ultrasonic surgical system according to the first embodiment of the present invention;
Fig. 5 is a block diagram of basic configuration of an output control unit;
Fig. 6 is a flowchart of various processing steps since an abnormality that occurs in the ultrasonic surgical system is detected until a processing for prohibiting the driving of this ultrasonic surgical system is performed;
Fig. 7 is a flowchart for detailed explanation of the respective processing steps until a before-driving-time abnormality that occurs in the ultrasonic surgical system is detected;
Fig. 8 is a flowchart for detailed explanation of the respective processing steps until a driving-preparation-time abnormality that occurs in the ultrasonic surgical system is detected;
Fig. 9 is a flowchart for detailed explanation of the respective processing steps until a driving-time abnormality that occurs in the ultrasonic surgical system is detected;
Fig. 10 is a flowchart for detailed explanation of the respective processing steps until an abnormality determination processing is performed in a test mode and an ultrasonic vibrator that has been stopped is permitted to be driven again;
Fig. 11 is a block diagram of basic configuration of an ultrasonic surgical system according to a second embodiment of the present invention;
Fig. 12 is a typical view of a state in which a jaw is closed relative to a probe;
Fig. 13 is a typical view of a state in which a jaw is closed relative to a probe according to a modification of the second embodiment; and
Fig. 14 is a flowchart of respective processing steps of prohibiting output of an ultrasonic vibration to the probe if it is detected that a short-circuit between the probe and the jaw occurs.

### DETAILED DESCRIPTION

Exemplary embodiments of an ultrasonic surgical system, and an abnormality detection method and an abnormality detection program for the ultrasonic surgical system according to the present invention will be explained hereinafter with reference to the accompanying drawings. A scissors type ultrasonic surgical system which incises a treatment target such as a biological tissue will be explained hereinafter as an ultrasonic surgical system according to the present invention.

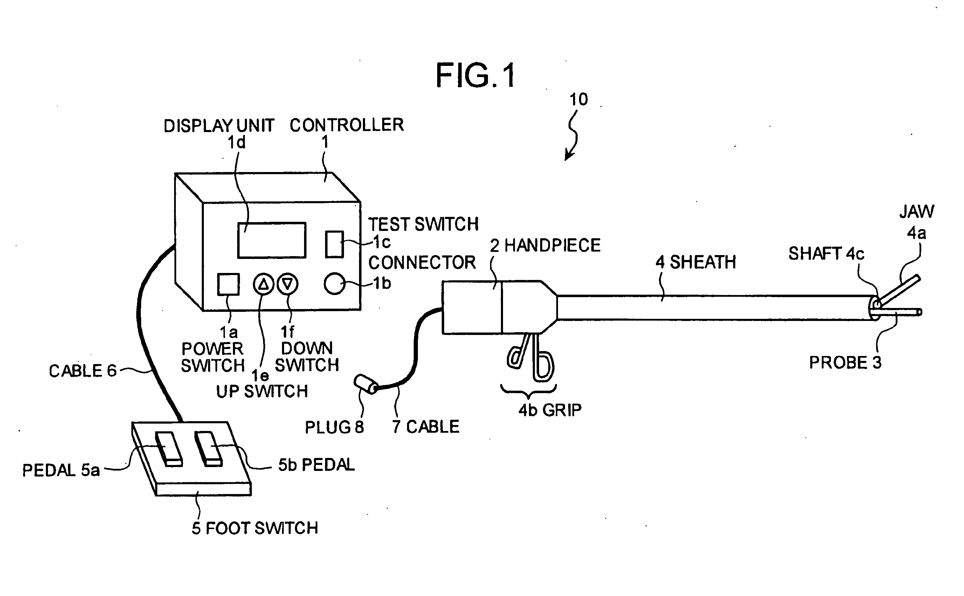

Fig. 1 is a typical view of the schematic configuration of the ultrasonic surgical system according to a first embodiment of the present invention. In Fig. 1, the ultrasonic surgical system 10 includes a controller main unit (hereinafter, "controller") 1, a handpiece 2, a probe 3, a sheath 4, and a foot switch 5. The controller 1 includes a power switch 1a, a connector 1b, a test switch 1c, a display unit 1d, an up switch 1e, and a down switch 1f. The handpiece 2 includes an ultrasonic vibrator (not shown) therein. The probe 3 is screwed with this ultrasonic vibrator. The probe 3 is inserted into the sheath 4, and the sheath 4 is detachably connected to the handpiece 2. If so, the sheath 4 covers the probe 3 for carrying out a medical treatment to a treatment target so as to expose a tip end of the probe 3 from a tip end of the sheath 4. The foot switch 5 includes pedals 5a and 5b, and is electrically connected to the controller 1 through a cable 6. The handpiece 2 includes a cable 7 having a plug 8 provided on one end. An other end of the cable 7 is electrically connected to the ultrasonic vibrator included in the handpiece 2. If the plug 8 is connected to the connector 1b in the controller 1, the handpiece 2 is electrically connected to the controller 1.

If the power switch 1a is operated to turn on the controller 1, the controller 1 acquires output setting information on the ultrasonic vibrator included in the handpiece 2 based on an instruction input from the pedal 5a or 5b of the foot switch 5, or controls the ultrasonic vibrator to be driven based on the acquired output setting information, and outputs a desired ultrasonic vibration to the ultrasonic vibrator. The controller 1 can adjust the output setting information on the ultrasonic vibrator included in the handpiece 2 by operating the up switch 1e or the down switch 1f.

The ultrasonic vibrator included in the handpiece 2 outputs the desired ultrasonic vibration to the probe 3 while being controlled to be driven by the controller 1. The probe 3, which is made of titanium or a titanium alloy, transmits the ultrasonic vibration output from the ultrasonic vibrator of the handpiece 2 to the tip end of the probe 3.

The sheath 4 includes a jaw 4a, a grip 4b, and a shaft 4c. The jaw 4a opens and closes the tip end of the probe about the shaft 4c by operating the grip 4b, and presses the treatment target on the tip end of the probe 3. If the desired ultrasonic vibration is transmitted to the tip end of the probe 3, then the jaw 4a performs an opening or closing operation based on the operation of the grip 4b, presses the treatment target on the tip end of the probe 3, and transmits the ultrasonic vibration to the treatment target. The medical treatment such as incision to the treatment target is thereby performed. In other words, the jaw 4a acts as a pressing unit that presses the treatment target on the tip end of the probe 3.

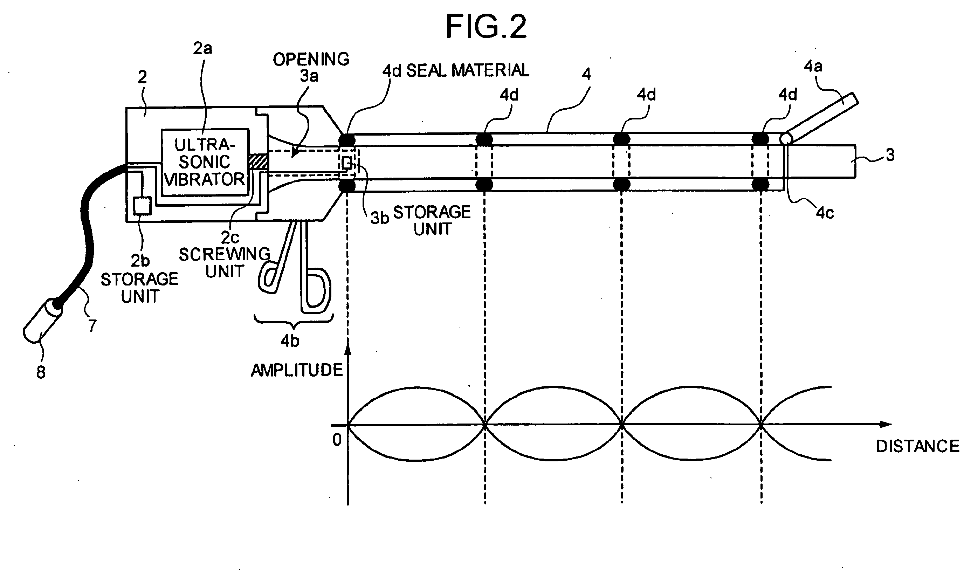

An arrangement state of the handpiece 2, the probe 3, and the sheath 4 will next be explained in detail. Fig. 2 is a typical view of the connection state between the ultrasonic vibrator included in the handpiece 2 and the probe 3 and between the sheath 4 into which the probe 3 is inserted and the handpiece 2. Fig. 2 also depicts a waveform of the ultrasonic vibration transmitted by the probe 3.

In Fig. 2, the handpiece 2 includes therein an ultrasonic vibrator 2a and a storage unit 2b. The ultrasonic vibrator 2a is screwed with the probe 3 by a screwing unit 2c. The probe 3 has an opening 3a formed near the screwing unit 2c, and a storage unit 3b arranged in the opening 3a. The ultrasonic vibrator 2a and the storage unit 2b are electrically connected to the cable 7. If the probe 3 is screwed with the ultrasonic vibrator 2a, the storage unit 3b is electrically connected to the cable 7 through a wiring provided at the handpiece 2. Accordingly, if the plug 8 is connected to the connector 1b of the controller 1 through the cable 7, then the ultrasonic vibrator 2a and the storage unit 2b are electrically connected to the controller 1 through the cable 7 and the storage unit 3b is electrically connected to the controller 1 through the handpiece 2 and the cable 7. If the ultrasonic vibrator 2a, which is made of piezoelectric ceramic such as PZT, is electrically connected to the controller 1, the ultrasonic vibrator 2a is controlled to be driven by the controller 1. By controlling the ultrasonic vibrator 2a to be driven by the controller 1, the ultrasonic vibrator 2a can output the desired ultrasonic vibration to the probe 3 through the screwing unit 2c.

The sheath 4, into which the probe 3 is inserted, is connected to the handpiece 2. If so, then a desired number of seal materials 4d are arranged at desired positions of an outer wall of the probe 3, respectively, and the probe 3 is detachably supported by the sheath 4 through the seal materials 4d. In this state, if the ultrasonic vibrator 2a outputs the ultrasonic vibration to the probe 3 through the screwing unit 2c, a longitudinal wave (standing wave) corresponding to this ultrasonic vibration occurs in the probe 3. As shown in Fig. 2, a phase of a waveform of the standing wave occurring in the probe 3 corresponds to a distance between the probe 3 near the screwing unit 2c of and the tip end of the probe 3, i.e., a position of the wave on the probe 3. The standing wave occurring in the probe 3 possesses a knot at a position at which the probe 3 is supported by the sheath 4 through the seal materials 4d and a belly on the tip end of the probe 3 for performing the medical treatment to the treatment target. Namely, the seal materials 4d are arranged so that this standing wave possesses the belly on the tip end of the probe 3, whereby the probe 3 can ensure transmitting the desired ultrasonic vibration to the tip end of the probe 3.

The number of seal materials 4d may be set so as to be able to ensure that the probe 3 is supported by the sheath 4. Desirably, the number of seal materials 4d is set so that the seal materials 4d are arranged equidistantly. If so setting, phenomena such as a bending and a shake of the probe 3 resulting from the ultrasonic vibration can be suppressed, thereby making it possible to reduce a load on the probe 3 and to stably transmit the ultrasonic vibration. O-rings or the like made of various resin such as silicon or rubber can be used as the seal materials 4d.

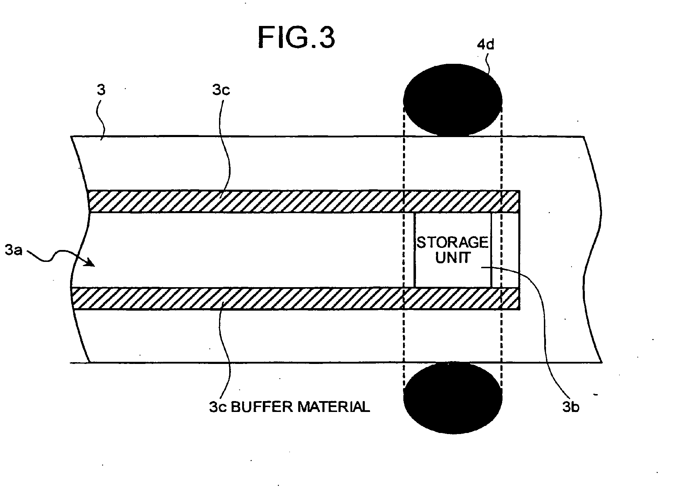

Fig. 3 is a longitudinal sectional, typical view of an arrangement state of the storage unit 3b arranged in the opening 3a of the probe 3. In Fig. 3, the storage unit 3b is arranged in the opening 3a located on inner peripheries of the seal materials 4d in contact with the outer wall of the probe 3, and fixed to an inner wall of the probe 3 through a buffer 3c. Namely, a part of the probe 3 in which the storage unit 3b is arranged corresponds to the knot of the standing wave occurring in the probe 3.

In the probe 3 to which the ultrasonic vibration is transmitted, a displacement quantity due to the ultrasonic vibration is minimum at the position of the knot of the standing wave that occurs in the probe 3, and a stress applied by the ultrasonic vibration most concentrates on the position of the knot. The buffer 3c is made of an elastic body such as rubber or arbitrary resin such as vinyl resin or urethane resin solely or a combination of the elastic body and the arbitrary resin. The buffer 3c fixes the storage unit 3b to the inner wall of the probe 3, and buffers concentration of the stress applied by the ultrasonic vibration on the storage unit 3b. Further, a wiring (not shown) for electrically connecting the storage unit 3b to the handpiece 2 is preferably provided on the buffer 3c. If so providing, it is possible to reduce the stress applied to this wiring and a malfunction such as disconnection can be prevented. If this wiring or the storage unit 3b is realized by a flexible substrate made of a resin tape or the like, it is possible to further relax the stress applied on this wiring or the storage unit 3b. The storage unit 3b is preferably arranged on a plane including a central axis of a cross section of the probe 3. If so arranging, the phenomena such as the binding and the shake of the probe 3 caused by the ultrasonic vibration can be suppressed and the load on the probe 3 can be reduced.

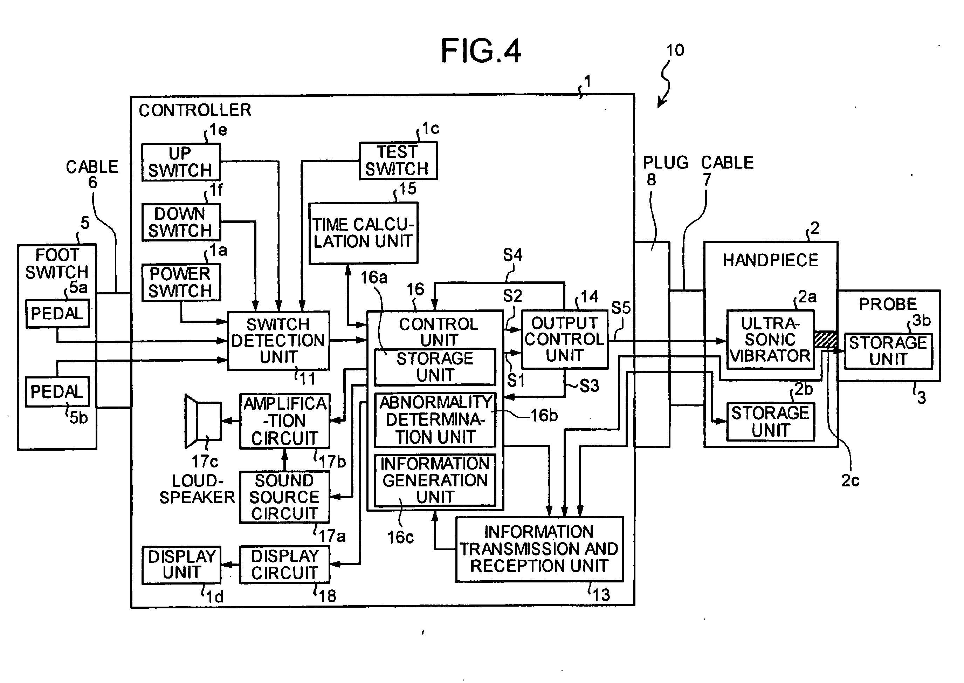

The basic configuration of the ultrasonic surgical system according to the first embodiment of the present invention will be explained in detail. Fig. 4 is a block diagram of the basic configuration of the ultrasonic surgical system 10. In Fig. 4, the ultrasonic surgical system 10 includes the controller 1, the foot switch 5 electrically connected to the controller 1 through the cable 6, the handpiece 2 electrically connected to the controller 1 through the cable 7 if the plug 8 is connected to the connector 1b, and the probe 3 screwed with the ultrasonic vibrator 2a by the screwing unit 2c as explained above.

The controller 1 includes the power switch 1a, the test switch 1c, the display unit 1d, the up switch 1e, and the down switch 1f as explained. The controller 1 also includes a switch detection unit 11, an information transmission and reception unit 13, an output control unit 14, a time calculation unit 15, a control unit 16, a sound source circuit 17a, an amplification circuit 17b, a loudspeaker 17c, and a display circuit 18. The pedals 5a and 5b, the power switch 1a, the test switch 1c, the up switch 1e, and the down switch 1f are electrically connected to the switch detection unit 11. The control unit 16 controls the information transmission and reception unit 13, the output control unit 14, the sound source circuit 17a, the amplification circuit 17b, and the display circuit 18. The switch detection unit 11 is electrically connected to the control unit 16. The information transmission and reception unit 13 is electrically connected to the storage units 2b and 3b. The output control unit 14 is electrically connected to the ultrasonic vibrator 2a. It is noted, however, that the electrical connection between the output control unit 14 and the ultrasonic vibrator 2a is held through a parallel coil (not shown) of the output control unit 14 so as to isolate a control potential of the ultrasonic surgical system 10 from a potential of a patient. The sound source circuit 17a is electrically connected to the amplification circuit 17b, and the amplification circuit 17b is electrically connected to the loudspeaker 17c. The display circuit 18 is electrically connected to the display unit 1d.

The switch detection unit 11 is set to constantly read information of switch-on or switch-off input from the power switch 1a. If receiving the switch-on information from the power switch 1a, the switch detection unit 11 is set to constantly read information of switch-on or switch-off input from the pedal 5a or 5b, the test switch 1c, the up switch 1e, or the down switch 1f. If the switch detection unit 11 inputs the switch-on information from the power switch 1a, that is, if the controller 1 is turned on by the operation of the power switch 1a, then the switch detection unit 11 detects that the power switch 1a is in an ON-state, and then transmits an instruction signal (i.e., an information read instruction signal) for reading the output control information and the determination criterion information stored in the storage units 2b and 2b to the control unit 16. If inputting the switch-on information from the pedal 5a or 5b, the switch detection unit 11 transmits a signal for starting a predetermined control corresponding to this information to the control unit 16. If inputting the switch-off information from the pedal 5a or 5b, the switch detection unit 11 transmits a signal for stopping the predetermined control corresponding to this information to the control unit 16. It is assumed that the switch-on information input from the pedal 5a corresponds to an output setting control for detecting the resonance frequency of the ultrasonic vibrator 2a to which the probe 3 is connected, and for setting a driving current and a driving voltage. It is also assumed that the switch-on information input from the pedal 5b corresponds to a driving control over the ultrasonic vibrator 2a (a vibrator driving control) based on the detected resonance frequency and the driving current and the driving voltage thus set. If so, then the switch detection unit 11 transmits the instruction signal (setting start instruction signal) for starting the output setting control to the control unit 16 based on the switch-on information input from the pedal 5a, and transmits the instruction signal (setting stop instruction signal) for stopping the output setting control to the control unit 16 based on the switch-off information input from the pedal 5a. Further, the switch detection unit 11 transmits the instruction signal (driving start instruction signal) for starting the vibrator driving control to the control unit 16 based on the switch-on information input from the pedal 5b, and transmits the instruction signal (driving stop instruction signal) for stopping the vibrator driving control to the control unit 16 based on the switch-off information input from the pedal 5b.

If inputting the switch-on information from the test switch 1c, the switch detection unit 11 transmits an instruction signal (a test mode switchover signal) for switching over a control mode in which the control unit 16 exercises control, to a test mode for testing whether an abnormality occurs in the ultrasonic surgical system 10 to the control unit 16. If inputting the switch-off information from the test switch 1c, the switch detection unit 11 transmits an instruction signal (a driving mode switchover signal) for switching over the control mode for the control unit 16 to a driving mode for exercising the output setting control or the vibrator driving control to the control unit 16. If inputting the switch-on information from the up switch 1e, the switch detection unit 11 transmits an instruction signal (a setting increase instruction signal) for increasing an output set value such as the driving current or the driving voltage by predetermined numeric values to the control unit 16. If inputting the switch-on information from the down switch 1f, the switch detection unit 11 transmits an instruction signal (a setting decrease instruction signal) for decreasing the output set value such as the driving current or the driving voltage by predetermined numeric values to the control unit 16. If receiving the switch-on information from the up switch 1e and the switch-on information from the down switch 1f simultaneously, the switch detection unit 11 transmits an instruction signal (a sound setting start signal) for starting a volume setting or a tone setting of an output sound output from the loudspeaker 17c to the control unit 16.

If detecting that both the up switch 1e and the down switch 1f have been in switch ON-states for a predetermined time (e.g., about one second) or more, then the switch detection unit 11 preferably recognizes that the unit 11 simultaneously receives the switch-on information from the up switch 1e and the switch-on information from the down switch 1f, and transmits a sound setting instruction signal to the control unit 16. By doing so, the respective operations for changing the output set value and setting the output sound can be clearly distinguished from one another and it is possible to ensure performing the respective operations.

If the switch detection unit 11 transmits the information read instruction signal to the control unit 16, the information transmission and reception unit 13 reads the output control information and the determination criterion information from the storage units 2b and 3b under control of the control unit 16. Specifically, the information transmission and reception unit 13 reads probe output control information and first determination criterion information stored in the storage unit 3b and vibrator output control information and second determination criterion information stored in the storage unit 2b. The information transmission and reception unit 13 converts the read probe output control information, vibrator output control information, first determination criterion information, and second determination criterion information into predetermined digital codes, respectively, and transmits the respective pieces of information converted into the predetermined digital codes to the control unit 16. Thus, the control unit 16 accomplishes an information read processing for reading the probe output control information and the first determination criterion information stored in the storage unit 3b, and the vibrator output control information and the second determination criterion information stored in the storage unit 2b. Further, if abnormality history information on a history of an abnormality that occurs in the ultrasonic surgical system 10 is stored in the storage unit 3b as the first determination criterion information, and stored in the storage unit 2b as the second determination criterion information, then the information transmission and reception unit 13 reads the abnormality history information from the storage unit 3b as the first determination criterion information or reads the abnormality history information from the storage unit 2b as the second determination criterion information under control of the control unit 16.

The output control information includes probe output control information serving as parameters for ultrasonic output characteristics of the probe 3 and vibrator output control information serving as parameters for ultrasonic characteristics of the ultrasonic vibrator 2a. The probe output control information includes, for example, a driving frequency, an amplitude magnification factor, a boosting ratio, and a rated voltage of the probe 3. The vibrator output control information includes, for example, a driving frequency, a current amplitude ratio, a boosting ratio, and a rated voltage of the ultrasonic vibrator 2a. The driving frequency is a frequency parameter corresponding to a reference frequency at which a frequency sweep processing for detecting a resonance frequency is performed. The current amplitude ratio and the amplitude magnification factor are operation parameters for operating and outputting a driving current parameter for setting a driving current at which an ultrasonic vibration having a desired amplitude is output. The boosting ratio is a current control parameter for setting a driving voltage dependent on impedance characteristics of the probe 3 and the ultrasonic vibrator 2a. The rated voltage is a parameter for setting a maximum output voltage of a control signal for controlling driving of the ultrasonic vibrator 2a.

The determination criterion information includes the first determination criterion information that is a criterion as to whether an abnormality resulting from the probe 3 occurs in the ultrasonic surgical system 10, and the second determination criterion information that is a criterion as to whether an abnormality resulting from the handpiece 2 occurs in the ultrasonic surgical system 10. The first determination criterion information includes, for example, a first accumulated output time, a first output control time, a frequency upper limit and a frequency lower limit, a nominal frequency and a frequency deviation limit, a driving voltage upper limit, or a current upper limit and a current lower limit. The second determination criterion information includes, for example, a second accumulated output time, a second output control time, a frequency upper limit and an frequency lower limit, a nominal frequency and a frequency deviation limit, a driving voltage upper limit, a driving current upper limit and a lower limit driving current, or a capacitance limit. The frequency upper limit and the frequency lower limit are values for limiting a sweep range of the driving frequency at which the frequency sweep processing is performed. The nominal frequency corresponds to the resonance frequency of the ultrasonic vibrator 2a to which the probe 3 is connected. The frequency deviation limit is a value for limiting an absolute value of a deviation of the resonance frequency detected by the frequency sweep processing from the nominal frequency. The driving voltage upper limit is a criterion for setting an upper limit of the driving voltage and is a criterion about an abnormality resulting from an overload on the probe 3 or the handpiece 2. The driving current upper limit is a criterion for setting an upper limit of the driving current and is a criterion about an abnormality resulting from an excessive output of the probe 3 or the handpiece 2. The lower limit driving current is a criterion for setting a lower limit of the driving current and is a criterion about an abnormality resulting from an overload on the probe 3 or the handpiece 2. The capacitance limit is a value for limiting a capacitance of the handpiece 2 dependent on a temperature change of the handpiece 2. The first accumulated output time is an accumulated time of outputting the ultrasonic vibration to the probe 3. The first output limit time is a limit to the first accumulated output time. The second accumulated output time is an accumulated time for which the ultrasonic vibrator 2a included in the handpiece 2 outputs the ultrasonic vibration. The second output limit time is a limit to the second accumulated output time.

It is assumed that the information transmission and reception unit 13 receives the abnormality history information, first accumulated time information corresponding to the first accumulated output time, or second accumulated time information corresponding to the second accumulated output time from the control unit. If so, the information transmission and reception unit 13 stores the received abnormality history information in the storage unit 3b as the first determination criterion information, stores the received abnormality history information in the storage unit 2b as the second determination criterion information, stores the received first accumulated time information in the storage unit 3b as the first determination criterion information, or stores the received second accumulated time information in the storage unit 2b as the second determination criterion information under control of the control unit 16. Accordingly, the control unit 16 can store the abnormality history information in the storage unit 2b or 3b, store the first accumulated time information in the storage unit 3b, or store the received second accumulated time information in the storage unit 2b. The control unit 16 can also update the abnormality history information already stored in the storage unit 2b or 3b, update the first accumulated time information already stored in the storage unit 3b, or update the second accumulated time information already stored in the storage unit 2b. If the control unit 16 thus updates the information and transmits the abnormality history information indicating that no abnormality occurs in the ultrasonic surgical system 10 to the storage unit 2b or 3b through the information transmission and reception unit 13, the control unit 16 can erase the abnormality history information stored in the storage unit 2b or 3b.

Reprogrammable nonvolatile memories such as erasable and programmable read only memory (EPROM) or electrically erasable programmable read only memory (EEPROM) can be employed as the storage units 2b and 3b. It is desirable that transmission and reception of various pieces of information between the storage units 2b or 3b and the information transmission and reception unit 13 are held by serial communication. By using the serial communication, the abnormality history information stored in the storage unit 2b or 3b can be erased.

The output control unit 14 is realized by a digital phase synchronized circuit including a direct digital synthesizer (DDS) or the like. The output control unit 14 performs the frequency sweep processing based on a reference frequency signal S1 output from the control unit 16, detects the resonance frequency of the ultrasonic vibrator 2a to which the probe 3 is connected, and exercises PLL control so that the ultrasonic vibrator 2a outputs the ultrasonic vibration at the resonance frequency or the near-resonance frequency. In addition, the output control unit 14 sets the driving current and the driving voltage based on a current and voltage setting signal S2 output from the control unit 16, and exercises a constant-current control so that a current of a driving signal for supplying an electric energy to the ultrasonic vibrator 2a is equal to the desired driving current. The output control unit 14 transmits a driving signal S5 including the set driving current and driving voltage and oscillating at the detected resonance frequency or the near-resonance frequency to the ultrasonic vibrator 2a. The output control unit 14 can thereby control the driving of the ultrasonic vibrator 2a so as to output the ultrasonic vibration having the desired amplitude at the resonance frequency.

The output control unit 14 detects an output frequency obtained by the frequency sweep processing, transmits a detected frequency signal S3 corresponding to the output frequency to the control unit 16, detects the driving current and the driving voltage set based on the current and voltage setting signal S2, and transmits a detected current and voltage signal S4 corresponding to the detected driving current and driving voltage to the control unit 16. It is noted that the output frequency is a frequency obtained by raising or lowering the driving frequency so as to detect or follow up the resonance frequency if the output control unit 14 performs the frequency sweep processing with the driving frequency used as the resonance frequency. Therefore, if the output control unit 14 exercises the PLL control, the output frequency corresponds to the resonance frequency or the near-resonance frequency of the ultrasonic vibrator 2a.

The time calculation unit 15 is realized while including a timer function of calculating an output time at which the ultrasonic vibrator 2a outputs the ultrasonic vibration to the probe 3. The time calculation unit 15 calculates the output time at which the ultrasonic vibrator 2a outputs the ultrasonic vibration to the probe 3 if the control unit 16 exercises the output setting control or vibrator driving control to the output control unit 14. For instance, if the control unit 16 receives the setting start instruction signal or the driving start instruction signal from the switch detection unit 11, the time calculation unit 15 starts an output time calculation processing. If the control unit 16 receives the setting stop instruction signal or the driving stop instruction signal from the switch detection unit 11, or stops driving the ultrasonic vibrator 2a due to occurrence of the abnormality, the time calculation unit 15 stops the output time calculation processing. Thereafter, the time calculation unit 15 transmits output time information corresponding to the output time calculated from the start to the end of this time calculation processing to the control unit 16. In the test mode, the time calculation unit 15 may calculate the output time at which the ultrasonic vibrator 2a outputs the ultrasonic vibration to the probe 3, and may transmit the calculated output time to the control unit 16 as the output time information.

The control unit 16 is realized by employing a storage unit 16a including a read only memory (ROM) that stores various pieces of data such as a processing program and a random access memory (RAM) that temporarily stores various pieces or information such as the output setting information and the determination criterion information, a central processing unit (CPU) that executes the processing program stored in the ROM, and the like. The control unit 16 includes an abnormality determination unit 16b and an information generation unit 16c. The CPU included in the control unit 16 enables respective processings to be explained later to be performed by the control unit 16, the abnormality determination unit 16b, and the information generation unit 16c, and realizes respective functions of the ultrasonic surgical system 10 by reading the processing program stored in a ROM included in the storage unit 16a and then executing the processing program.

If receiving the information read instruction signal from the switch detection unit 11, the control unit 16 controls the information transmission and reception unit 13 to read the output control information and the determination criterion information from the storage units 2b and 3b. If the control unit thus reads the output control information and the determination criterion information from the storage units 2b and 3b through the information transmission and reception unit 13, then the information generation unit 16c calculates and outputs the driving frequency, the driving current parameter, the boosting ratio, or the like based on the probe output control information and the vibrator output control information read as the output control information, and the control unit 16 stores the respective parameters thus obtained in the storage unit 16a as the output setting information. The control unit 16 also stores the read determination criterion information in the storage unit 16a.

As explained above, the output setting information is information for exercising a driving control so as to output the desired ultrasonic vibration from the ultrasonic vibrator 2a to the probe 3. The output setting information includes the driving frequency corresponding to a reference frequency of the frequency sweep processing for detecting the resonance frequency of the ultrasonic vibrator 2a to which the probe 3 is connected, the driving current parameter for setting the driving current for outputting the ultrasonic vibration having the desired amplitude from the ultrasonic vibrator 2a, the boosting ratio for setting the driving voltage for exercising the constant-current control to keep the set driving current constant, or the like.

If receiving the setting start instruction signal from the switch detection unit 11, then the control unit 16 controls the output control unit 14 based on the output setting information stored in the storage unit 16a, and performs the frequency sweep processing for detecting the resonance frequency of the ultrasonic vibrator 2a to which the probe 3 is connected and sets the driving current and the driving voltage. If so, the control unit 16 transmits the reference frequency signal S1 corresponding to the driving frequency stored in the storage unit 16a and the current and voltage setting signal S2 corresponding to the driving current parameter and the boosting ratio to the output control unit 14. The control unit 16 then receives the detected frequency signal S3 corresponding to the output frequency obtained by the frequency sweep processing performed by the output control unit 14, and stores the frequency information corresponding to the received detected frequency signal S3 in the storage unit 16a as the output setting information. Further, the control unit 16 receives the detected current and voltage signal S4 corresponding to the driving current and the driving voltage set by the output control unit 14, and stores the respective information on the driving current and the driving voltage corresponding to the received detected current and voltage signal S4 in the storage unit 16a as the output setting information. If so, the control unit 16 can use the frequency information and the respective information on the driving current and the driving voltage as the output setting information. If receiving the setting stop instruction signal from the switch detection unit 11, the control unit 16 stops the output setting control. If the output control unit 14 exercises the PLL control and sets the driving current and the driving voltage, the control unit 16 can receive a vibrator driving control start instruction by the driving start instruction signal.

Further, if receiving the driving start instruction signal from the switch detection unit 11, the control unit 16 exercises the vibrator driving control over the output control unit 14 based on the output setting information stored in the storage unit 16a. As a result, the ultrasonic vibrator 2a is controlled to be driven to output the ultrasonic vibrator having the desired amplitude to the probe 3. In this driving control, the ultrasonic vibration output from the ultrasonic vibrator 2a is transmitted to the probe 3 through the screwing unit 2c. The operator can, therefore, carry out the medical treatment such as incision to the treatment target using the probe 3 to which the ultrasonic vibration having the desired amplitude is transmitted. Thereafter, if receiving the driving stop instruction signal from the switch detection unit 11, the control unit 16 controls the output control unit 14 to stop driving the ultrasonic vibrator 2a. If the control unit 16 receives the driving stop instruction signal to thereby stop driving the ultrasonic vibrator 2a, then the information generation unit 16c may generate the output control information corresponding to latest output setting information stored in the storage unit 16a, and may transmit the generated output control information to the storage units 2b and 3b through the information transmission and reception unit 13. By doing so, the control unit 16 can update the output control information stored in the storage units 2b and 3b in advance.

If exercising the output setting control or the vibrator driving control to thereby output the ultrasonic vibration from the ultrasonic vibrator 2a, the control unit 16 can inform the operator that the ultrasonic vibrator 2a is outputting the ultrasonic vibration using an output sound or an output display. If so, the control unit 16 may transmit a predetermined display instruction signal to the display circuit 18, and may output the output display indicating that the ultrasonic vibrator 2a is outputting the ultrasonic vibration to the display unit 1d. Further, the control unit 16 may transmit a predetermined sound instruction signal to the sound source circuit 17a and output the output sound indicating that the ultrasonic vibrator 2a is outputting the ultrasonic vibration from the loudspeaker 17c. At this moment, if receiving the sound setting start signal from the switch detection unit 11, the control unit 16 switches over an output adjustment mode for increasing or decreasing the output set value by predetermined numeric values to a sound adjustment mode for making a volume setting or a tone setting of the output sound. In the sound adjustment mode, the control unit 16 transmits an instruction signal for changing the tone to the sound source circuit 17a or transmits an instruction signal for increasing the volume to the amplification circuit 17b if receiving the setting increase instruction signal. Likewise, if receiving the setting decrease instruction signal, the control unit 16 transmits the instruction signal for changing the tone to the sound source circuit 17a or transmits an instruction signal for reducing the volume to the amplification circuit 17b. As a result, the sound source circuit 17a is set to output a desired tone or the amplification circuit 17b is set to output the output sound having a desired volume. Accordingly, the sound source circuit 17a transmits a sound source signal corresponding to a sound source of the desired tone to the amplification circuit 17b. The amplification circuit 17b mixes or amplifies the volume-relating instruction signal received from the control unit 16 and the sound source signal received from the sound source circuit 17a, and transmits a signal corresponding to the desired volume and the desired tone to the loudspeaker 17c. The loudspeaker 17c outputs the output sound of the desired volume and the desired tone based on the signal received from the amplification circuit 17b. The control unit 16 may control the display circuit 18 so as to display setting information on the tone or the volume of the output sound output from the loudspeaker 17c on the display unit 1d.

Thereafter, if the switch detection unit 11 detects the switch-on information output from the up switch 1e and the switch-on information output from the down switch 1f simultaneously again, or if the switch detection unit 11 does not detect the switch-on information output from the up switch 1e or the switch-on information from the down switch 1f for a predetermined specified time (e.g., about ten seconds) or more, the control unit 16 switches over the sound adjustment mode to the output adjustment mode. In the output adjustment mode, the control unit 16 increases the output set value and controls the display circuit 18 to display information corresponding to the increased output set value on the display unit 1d if receiving the setting increase instruction signal. Likewise, if receiving the setting decrease instruction signal, the control unit 16 decreases the output set value and controls the display circuit 18 to display information corresponding to the decreased output set value on the display unit 1d. The display unit 1d may output an output display including characters, symbols, alphanumeric characters, or the like solely or a combination thereof, or may output an output display using a light emitting diode (LED) for indicating the volume.

The abnormality determination unit 16b determines whether an abnormality (a before-driving-time abnormality) occurs in the ultrasonic surgical system 10 before the ultrasonic vibrator 2a is driven based on a result of the information read processing or the like. In addition, the abnormality determination unit 16b determines whether an abnormality (a driving-preparation-time abnormality) occurs in the ultrasonic surgical system 10 based on the determination criterion information read from the storage units 2b and 3b through the information transmission and reception unit 13 or the like if the control unit 16 exercises the output setting control. Further, the abnormality determination unit 16b determines whether an abnormality (a driving-time abnormality) occurs in the ultrasonic surgical system 10 based on this determination criterion information or the like if the control unit exercises the vibrator driving control. Examples of the before-driving-time abnormality include a connection abnormality that the connection between the probe 3 and the ultrasonic vibrator 2a or the connection between the handpiece 2 and the probe 3 is inappropriate, and a combination abnormality that the combination of the handpiece 2 and the probe 3 is inappropriate. Examples of the driving-preparation-time abnormality include a frequency abnormality that occurs due to the overload on the vibration system in the ultrasonic surgical system 10 resulting from the disconnection of the electric wiring at the handpiece 2, the damage of the probe 3, the adhesion of the biological tissue such as the blood to the probe 3, or the like. Examples of the driving-time abnormality include a driving current abnormality that occurs due to the excessive output of the ultrasonic vibration from the ultrasonic vibrator 2a or the overload resulting from the disconnection of the electric wiring at the handpiece 2, the damage of the probe 3, or the like, a driving voltage abnormality that the driving voltage is saturated due to the disconnection of the electric wiring at the handpiece 2, the damage of the probe 3, or the like, thereby making it difficult to supply the electric energy to the ultrasonic vibrator 2a, an accumulated time abnormality that the accumulated output time of the ultrasonic vibrator 2a or that of the probe 3 is equal to or longer than a predetermined limit time, a handpiece temperature abnormality that a temperature of the handpiece 2 excessively rises, a probe temperature abnormality that a temperature of the probe 3 excessively rises, and the frequency abnormality. Examples of a combination abnormality detected as the before-driving-time abnormality include an instance in which the abnormality history information on the handpiece 2 or the probe 3 is present, and an instance in which the accumulated output time of the ultrasonic vibrator 2a or that of the probe 3 is equal to or longer than the predetermined limit time.

If the abnormality determination unit 16b determines that the before-driving-time abnormality, the driving-preparation-time abnormality, or the driving-time abnormality occurs in the ultrasonic surgical system 10, the control unit 16 detects the before-driving-time abnormality, the driving-preparation-time abnormality, or the driving-time abnormality that occurs in the ultrasonic surgical system 10. If so, the control unit 16 stops driving the ultrasonic vibrator 2a, and prohibits reception of the instruction by the setting start instruction signal or the driving start instruction signal received from the switch detection unit 11. Accordingly, the control unit 16 prohibits the ultrasonic vibrator 2a from outputting the ultrasonic vibration, transmits the instruction signal for outputting the output sound corresponding to the detected abnormality to the sound source circuit 17a or the amplification circuit 17b, or transmits the instruction signal for outputting the output display corresponding to the detected abnormality to the display circuit 18. The control unit 16 thus notifies the operator of occurrence of the before-driving-time abnormality, the driving-preparation-time abnormality, or the driving-time abnormality to the ultrasonic surgical system 10 (the control unit 16 performs a driving prohibition processing).

As explained above, the information generation unit 16c generates and outputs the output setting information such as the driving frequency, the driving current parameter, or the boosting ratio based on the probe output control information and the vibrator output control information read as the output control information. If the abnormality determination unit 16b determines occurrence of the driving-preparation-time abnormality or the driving-time abnormality, the information generation unit 16c generates abnormality history information corresponding to occurrence of the driving-preparation-time abnormality or the driving-time abnormality to the ultrasonic surgical system 10. The control unit 16 stores the abnormality history information generated by the information generation unit 16c in the storage units 2b and 3b through the information transmission and reception unit 13. It is noted that the control unit 16 stores the abnormality history information generated by the information generation unit 16c according to types of abnormalities. For example, the control unit 16 stores the abnormality history information corresponding to the frequency abnormality, the driving current abnormality, or the driving voltage abnormality in the storage units 2b and 3b. The control unit 16 stores the abnormality history information corresponding to the accumulation time abnormality or the handpiece temperature abnormality of the ultrasonic vibrator 2a in the storage unit 2b. The control unit 16 stores the abnormality history information corresponding to the accumulated time abnormality or the probe temperature abnormality of the probe 3 in the storage unit 3b.

Further, the information generation unit 16c calculates and outputs an accumulated time for which the ultrasonic vibration is output to the probe 3 based on the output time information received from the time calculation unit 15 and the first accumulated time that is the first determination criterion information read from the storage unit 3b, and generates the first accumulated time information corresponding to the accumulated time thus obtained. If so, the control unit 16 stores the accumulated time calculated and output by the information generation unit 16c in the storage unit 16a as the latest first accumulated output time. If stopping the driving of the ultrasonic vibrator 2a, the control unit 16 transmits the first accumulated time information corresponding to the latest first accumulated output time to the storage unit 3b through the information transmission and reception unit 13, and stores the first accumulated time information in the storage unit 3b. Likewise, the information generation unit 16c calculates and outputs an accumulated time for which the ultrasonic vibrator 2a outputs the ultrasonic vibration based on the output time information received from the time calculation unit 15 and the second accumulated output time that is the second determination criterion information read from the storage unit 2b, and generates second accumulated time information corresponding to the accumulated time thus obtained. If so, the control unit 16 stores the accumulated time calculated and output by the information generation unit 16c in the storage unit 16a as the latest second accumulated output time. If stopping the driving of the ultrasonic vibrator 2a, the control unit 16 transmits the second accumulated time information corresponding to the latest second accumulated output time to the storage unit 3b through the information transmission and reception unit 13, and stores the second accumulated time information in the storage unit 3b.

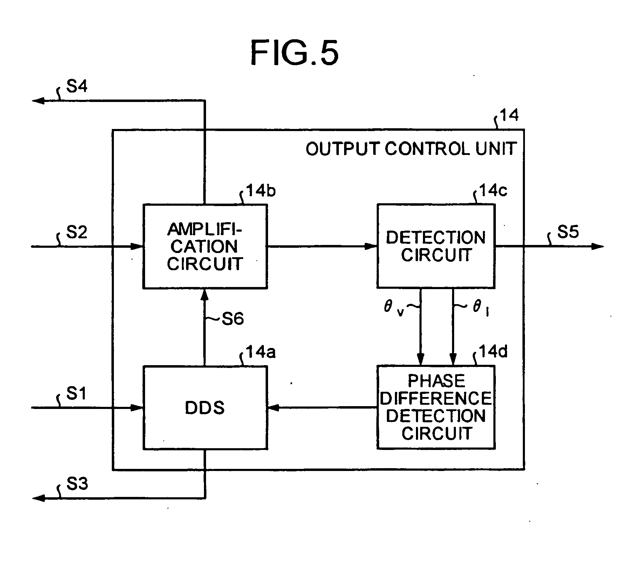

The configuration of the output control unit 14 will next be explained in detail. Fig. 5 is a block diagram of the basic configuration of the output control unit 14 in the ultrasonic surgical system 10 according to the first embodiment of the present invention. In Fig. 5, the output control unit 14 includes a DDS 14a, an amplification circuit 14b, a detection circuit 14c, and a phase difference detection circuit 14d. The DDS 14a is connected to the amplification circuit 14b and the phase difference detection circuit 14d. The detection circuit 14c is connected to the amplification circuit 14b and the phase difference detection circuit 14d. Thus, the DDS 14a, the amplification circuit 14b, the detection circuit 14c, and the phase difference detection circuit 14d form a loop circuit. Further, the DDS 14a and the amplification circuit 14b are connected to the control unit 16, and the detection circuit 14c is connected to the ultrasonic vibrator 2a.

The DDS 14a oscillates the signal at the driving frequency corresponding to the reference frequency signal S1 received from the control unit 16 and transmits the signal to the amplification circuit 14b right after the ultrasonic surgical system 10 is actuated. The amplification circuit 14b sets the driving current using the driving current parameter corresponding to the current and voltage setting signal S2 received from the control unit 16, and sets the driving voltage using the boosting ratio corresponding to the current and voltage setting signal S2. In setting the driving voltage, the amplification circuit 14b selects the boosting ratio so that the rated voltage serving as the output control information is the upper limit of the driving voltage, and makes setting of the driving voltage. Accordingly, the amplification circuit 14b exercises the constant-current control so that the current of the driving signal supplied to the ultrasonic vibrator 2a is equal to the driving current which is set using this driving current parameter. Thereafter, the amplification circuit 14 transmits the signal at the driving current and the driving voltage thus set and oscillated at the set frequency by the DDS 14a to the detection circuit 14c.

The detection circuit 14c detects a current phase and a voltage phase of the signal received from the amplification circuit 14b, and generates a current phase signal θ_{I} corresponding to the current phase and a voltage phase signal θ_{V} corresponding to the voltage phase. The detection circuit 14c transmits the current phase signal θ_{I} and the voltage phase signal θ_{V} thus generated to the phase difference detection circuit 14d, and supplies the driving signal S5 to the ultrasonic vibrator 2a as the signal received from the amplification circuit 14b. Therefore, the detection circuit 14c detects the current phase and the voltage phase of the driving signal S5 for driving the ultrasonic vibrator 2a.

The phase difference detection circuit 14d detects a phase difference between the current and the voltage of the driving signal S5 based on the current phase signal θ_{I} and the voltage phase signal θ_{V} received from the detection circuit 14c. The phase difference detection circuit 14d then generates a frequency control signal for controlling the driving frequency corresponding to the reference frequency signal S1 to be raised or lowered based on the detected phase difference, and transmits the generated frequency control signal to the DDS 14a. If so, the DDS 14a sets the driving frequency corresponding to the reference frequency signal S1 at the reference frequency, raises or lowers the driving frequency according to the frequency control signal received from the phase difference detection circuit 14d. As a result, the DDS 14a controls the driving frequency so as to set the phase difference detected by the phase difference detection circuit 14d at zero, oscillates the signal at the driving frequency controlled so that this phase difference is zero, and transmits an output frequency signal to the amplification circuit 14b as the signal oscillated at this driving frequency. Thus, the output control unit 14 accomplishes the frequency sweep processing for detecting the resonance frequency of the ultrasonic vibrator 2a to which the probe 3 is connected, and accomplishes the PLL control to control the ultrasonic vibrator 2a to output the ultrasonic vibration at the resonance frequency or the near-resonance frequency. The output control unit 14 can thereby transmit the driving signal S5 oscillated at the resonance frequency or the near-resonance frequency thus detected to the ultrasonic vibrator 2a, and can control the ultrasonic vibrator 2a to be driven to output the ultrasonic vibration having the desired amplitude at the resonance frequency.

Meanwhile, the DDS 14a detects the output frequency obtained by this frequency sweep processing, and transmits the detection frequency signal S3 corresponding to the output frequency to the control unit 16. The amplification circuit 14b detects the driving current and the driving voltage which are set based on the current and voltage setting signal S2, and transmits the detected current and voltage signal S4 corresponding to the detected driving current and driving voltage to the control unit 16. During the frequency sweep processing or the PLL control, the DDS 14a constantly detects the output frequency, and constantly transmits the detected frequency signal S3 corresponding to the detected output frequency to the control unit 16. During the constant-current control, the amplification circuit 14b constantly detects the driving current and the driving voltage, and constantly transmits the detected current and voltage signal S4 corresponding to the detected driving current and driving voltage to the control unit 16. As a result, the control unit 16 can detect the latest output frequency, the latest driving current, or the latest driving voltage detected by the output control unit 14.

The output control unit 14 may be realized by using an analog phase synchronized circuit including a phase comparator, a lowpass filter, a voltage control oscillator, and the like. Desirably, however, the output control unit 14 is realized by using the digital phase synchronized circuit. This is because if the analog phase synchronized circuit is used, the frequency characteristics of the circuit changes according to the temperature change or the like.

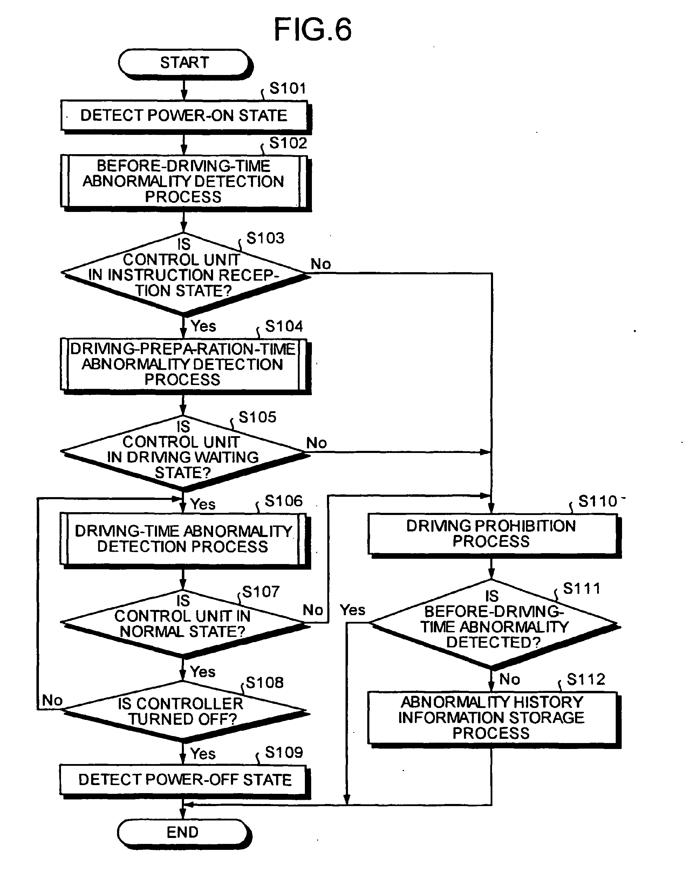

Respective processing steps since the control unit 16 detects that an abnormality occurs in the ultrasonic surgical system 10 until the driving prohibition processing is carried out to the ultrasonic surgical system 10 to which the abnormality occurs will next be explained in detail. Fig. 6 is a flowchart of the respective processing steps since the control unit 16 detects that an abnormality occurs in the ultrasonic surgical system 10 until the driving prohibition processing is carried out to the ultrasonic surgical system 10 to which the abnormality occurs. Referring to Fig. 6, if the controller 1 is turned on by the operation of the power switch 1a, then the switch detection unit 11 detects that the power switch 1a is in a switch-ON-state (power-ON state) (at step S101), and transmits the information read instruction signal to the control unit 16. The control unit 16 performs the information read processing if receiving the information read instruction signal from the switch detection unit 11. The abnormality determination unit 16b determines whether the before-driving-time abnormality occurs in the ultrasonic surgical system 10 based on a result of the information read processing or the determination criterion information read in the information read processing. If the abnormality determination unit 16b determines that the before-driving-time abnormality occurs in the ultrasonic surgical system 10, the control unit 16 detects the before-driving-time abnormality (at step S102). If the abnormality determination unit 16b determines that the before-driving-time abnormality does not occur in the ultrasonic surgical system 10, then the information generation unit 16c calculates and outputs the driving frequency, the driving current parameter, the boosting ratio, or the like based on the output control information read in the information read processing, and the control unit 16 stores the obtained parameter in the storage unit 16a as the output setting information. If so, the control unit 16 turns into a state (an instruction reception state) for receiving the output setting control instruction by the setting start instruction signal.

If the control unit 16 is in the instruction reception state ("Yes" in step S103) and receives the setting start instruction signal from the switch detection unit 11, then the control unit 16 exercises the output setting control over the output control unit 14, and performs the frequency sweep processing for detecting the resonance frequency of the ultrasonic vibrator 2a to which the probe 3 is connected. The abnormality determination unit 16b determines whether the driving-preparation-time abnormality occurs in the ultrasonic surgical system 10 based on the output frequency detected in the frequency sweep processing and the determination criterion information read in the information read processing. If the abnormality determination unit 16b determines that the driving-preparation-time abnormality occurs in the ultrasonic surgical system 10, the control unit detects the driving-preparation-time abnormality (at step S104). If the abnormality determination unit 16b determines that the driving-preparation-time abnormality does not occur in the ultrasonic surgical system 10, then the output control unit 14 accomplishes the frequency sweep processing to thereby detect the resonance frequency as explained above. In addition, the output control unit 14 exercises the PLL control so as to oscillate the ultrasonic vibration at the resonance frequency or the near-resonance frequency, sets the driving current and the driving voltage, and exercises the constant-current control so as to output the ultrasonic vibration having the desired amplitude. If so, the control unit 16 turns into a state (a driving waiting state) for receiving the vibrator driving control instruction by the driving start instruction signal.

If the control unit 16 is in the driving waiting state ("Yes" at step S105) and receives the driving start instruction signal from the switch detection unit 11, then the control unit 16 exercises the vibrator driving control over the output control unit 14 and drives the ultrasonic vibrator 2a to output the ultrasonic vibration having the desired amplitude to the probe 3. The abnormality determination unit 16b determines whether the driving-time abnormality occurs in the ultrasonic surgical system 10 based on the output frequency, the driving current, or the driving voltage detected by the output control unit 14 and the determination criterion information read in the information read processing. If the abnormality determination unit 16b determines that the driving-time abnormality occurs in the ultrasonic surgical system 10, the control unit 16 detects the driving-time abnormality (at step S106). If the abnormality determination unit 16b determines that the driving-time abnormality does not occur in the ultrasonic surgical system 10, the ultrasonic surgical system 10 is in a normal state in which no driving-time abnormality occurs in the system 10 ("Yes" at step S107). If so, the control unit 16 exercises the vibrator driving control over the output control unit 14 based on the instruction by the driving start instruction signal received from the switch detection unit 11. The output control unit 14 controls the ultrasonic vibrator 2a to be driven to output the ultrasonic vibration having the desired amplitude to the probe 3 under the control of the control unit 16. Thus, the ultrasonic vibration output from the ultrasonic vibrator 2a is transmitted to the probe 3 through the screwing unit 2c. The operator can, therefore, carry out the medical treatment such as incision to the treatment target using the probe 3 to which the ultrasonic vibration having the desired amplitude is transmitted. Thereafter, if the controller 1 is to be turned off ("Yes" at step S108), then the operator operates the power switch 1a and the switch-off information is input to the switch detection unit 11 from the power switch 1a. If so, the switch detection unit 11 detects that the power switch 1a is in a switch-OFF state (power-OFF state), and transmits the driving stop instruction signal to the control unit 16. The control unit 16 controls the output control unit 14 to stop driving the ultrasonic vibrator 2a based on the instruction by the driving stop instruction signal received from the switch detection unit 11. If the controller 1 is not to be turned off ("No" at step S108), the control unit 16 repeatedly executes the respective steps after step S106.

If the abnormality determination unit 16b determines that the before-driving-time abnormality occurs in the ultrasonic surgical system 10 and the control unit 16 is not in the instruction reception state ("No" at step S103), if the abnormality determination unit 16b determines that the driving-preparation-time abnormality occurs in the ultrasonic surgical system and the control unit 16 is not in the driving waiting state ("No" at step 105), or if the abnormality determination unit 16b determines that the driving-time abnormality occurs in the ultrasonic surgical system 10 and the control unit 16 is not in the normal state ("No" at step S107), then the control unit 16 performs the driving prohibition processing to prohibit the ultrasonic vibrator 2a from outputting the ultrasonic vibration, controls the output of the output sound or the output display corresponding to the detected abnormality, and notifies the operator that the before-driving-time abnormality, the driving-preparation-time abnormality, or the driving-time abnormality occurs in the ultrasonic surgical system 10 (at step S110). If the abnormality detected by the control unit 16 is not the before-driving-time abnormality ("No" at step S111), then the information generation unit 16c generates the abnormality history information corresponding to the detected abnormality, and the control unit 16 stores the abnormality history information generated by the information generation unit 16c in the storage units 2b and 3b as explained above (at step S112). The control unit 16 may perform the abnormality history information storage processing at step S112 either simultaneously with or before the driving prohibition processing at step S110. If the abnormality detected by the control unit 16 is the before-driving-time abnormality ("Yes" at step S111), the information generation unit 16c does not generates the abnormality history information corresponding to the before-driving-time abnormality. Namely, the control unit 16 does not store the abnormality history information corresponding to the before-driving-time abnormality in the storage units 2b and 3b.

If the control unit performs the driving prohibition processing at step S110, the operator can recognize occurrence of an abnormality to the ultrasonic surgical system 10 and carry out a predetermined abnormality processing to the ultrasonic surgical system 10. Further, the control unit 16 carries out an abnormality determination processing to be explained later to the ultrasonic surgical system 10 to which the operator carries out the abnormality processing. If checking that no abnormality occurs in the ultrasonic surgical system 10, the control unit 16 can permit the ultrasonic vibrator 2a the driving of which is stopped by the driving prohibition processing to be driven again.

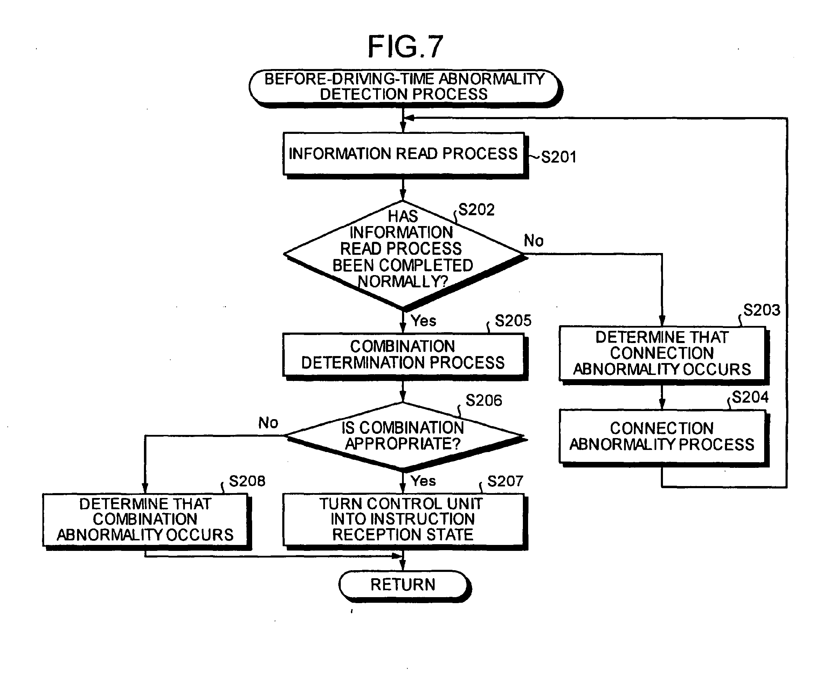

Fig. 7 is a flowchart for detailed explanation of respective processing steps until the control unit 16 detects the before-driving-time abnormality occurring in the ultrasonic surgical system 10 at step S102. Referring to Fig. 7, the control unit 16 performs the information read processing if receiving the information read instruction signal from the switch detection unit 11 (at step S201), and reads the output control information and the determination criterion information from the storage units 2b and 3b through the information transmission and reception unit 13. If the control unit 16 cannot read the probe output control information or the first determination criterion information from the storage unit 3b, or cannot read the vibrator output control information or the second determination criterion information from the storage unit 2b, and the control unit 16 cannot normally complete the information read processing at step S201 ("No" at step S202), then the abnormality determination unit 16b determines that the connection abnormality occurs in the ultrasonic surgical system 10 (at step S203) and the control unit 16 detects the connection abnormality occurring in the ultrasonic surgical system 10. If so, the control unit 16 performs a connection abnormality processing. Namely, the control unit 16 transmits the instruction signal for outputting the output sound corresponding to occurrence of the connection abnormality to the sound source circuit 17a or the amplification circuit 17b and transmits this output sound from the loudspeaker 17c, or transmits the instruction signal for outputting the output display corresponding to occurrence of the connection abnormality to the display circuit 18, and outputs this output display to the display unit 1d. Thus, the control unit 16 notifies the operator of occurrence of the connection abnormality to the ultrasonic surgical system 10 (at step S204). The control unit 16 then repeatedly executes the respective steps after step S201.

If the control unit 16 can read the probe output control information and the first determination criterion information from the storage unit 3b and the vibrator output control information and the second determination criterion information from the storage unit 2b, and can normally complete the information read processing at step S201 ("Yes" at step S202), then the abnormality determination unit 16b determines whether the combination of the handpiece 2 and the probe 3 is appropriate based on a result of comparison between the nominal frequency or the like in the first determination criterion information and the nominal frequency or the like in the second determination criterion information (at step S205). For example, if the nominal frequency in the first determination criterion information differs from that in the second determination criterion information, if the first accumulated output time is equal to or longer than the first output limit time, if the second accumulated output time is equal to or longer than the second output limit time, or the first determination criterion information or the second determination criterion information includes the abnormality history information, then the abnormality determination unit 16b determines that the combination of the handpiece 2 and the probe 3 is inappropriate ("No" at step S206), and determines that the combination abnormality occurs in the ultrasonic surgical system 10 (at step S208). If so, the control unit 16 detects the combination abnormality occurring in the ultrasonic surgical system 10.

Alternatively, the abnormality determination unit 16b may determine whether the combination of the handpiece 2 and the probe 3 is appropriate based on a result of comparison between the vibrator output control information read from the storage unit 2b and the probe output control information read from the storage unit 3b. In this alternative, the vibrator output control information and the probe output control information are used as the determination criterion information for determining whether the combination abnormality occurs in the ultrasonic surgical system 10. For instance, if the driving frequency of the vibrator output control information differs from that of the probe output control information, the abnormality determination unit 16b determines that the combination abnormality occurs in the ultrasonic surgical system 10.

If the abnormality determination unit 16b executes step S205 and determines that the combination of the handpiece 2 and the probe 3 is appropriate ("Yes" at step S206), the control unit 16 turns into the instruction reception state (at step S207). If so, the ultrasonic surgical system 10 is in a normal state in which no connection abnormality and no combination abnormality occur in the system 10.

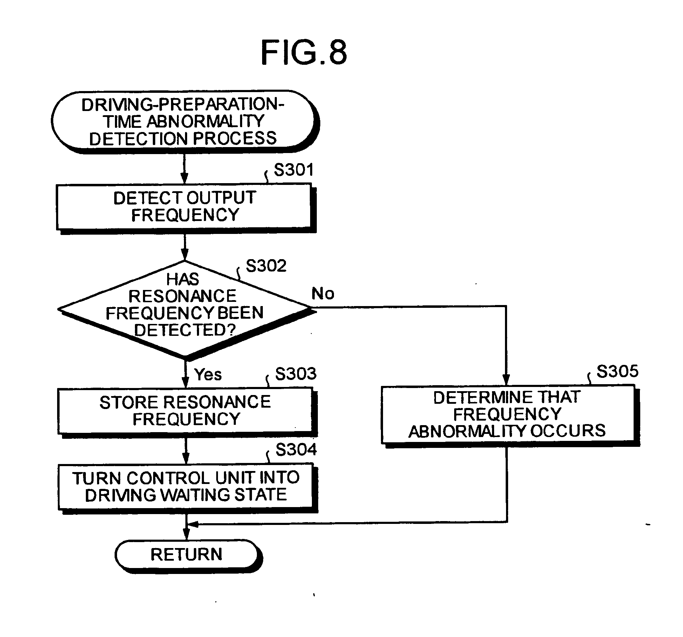

Fig. 8 is a flowchart for detailed explanation of respective steps until the control unit 16 detects the driving-preparation-time abnormality occurring in the ultrasonic surgical system 10 at step S104. Referring to Fig. 8, the control unit 16 exercises the output setting control over the output control unit 14 if receiving the setting start instruction signal from the switch detection unit 11. If so, the output control unit 14 performs the frequency sweep processing, sets the driving current and the driving voltage, and transmits the detected frequency signal S3 corresponding to the output frequency obtained in this frequency sweep processing and the detected current and voltage signal S4 corresponding to the driving current and the driving voltage thus set to the control unit 16 under control of the control unit 16. The control unit 16 detects the output frequency based on the detected frequency signal S3 received from the output control unit 14 (at step S301). It is noted, however, that if the output control unit 14 detects a resonance frequency Fr of the ultrasonic vibrator 2a, to which the probe 3 is connected, in the frequency sweep processing, then the control unit 16 detects the output frequency equal to the resonance frequency Fr based on the detected frequency signal S3 received from the output control unit 14.

The abnormality determination unit 16b constantly monitors the output frequency detected by the detection unit 16 based on the first determination criterion information or the second determination criterion information read from the storage units 2b and 3b in the information read processing. That is, the abnormality determination unit 16b determines whether the output frequency equal to the resonance frequency Fr is within a frequency range set by the frequency upper limit and the frequency lower limit in the first determination criterion information, and whether the output frequency equal to the resonance frequency Fr is within a frequency range set by the frequency upper limit and the frequency lower limit in the second determination criterion information. If the control unit 16 detects the output frequency equal to the resonance frequency Fr and the abnormality determination unit 16b determines that this output frequency is within the frequency range set by the first determination criterion information or the second determination criterion information, then the resonance frequency Fr detected by the output control unit 14 is within this frequency range, and the control unit 16 can detect the resonance frequency within this frequency range based on the detected frequency signal S3 received from the output control unit 14.

If the control unit 16 can detect the resonance frequency Fr within the frequency range ("Yes" at step S302), the control unit 16 stores the detected resonance frequency in the storage unit 16a as the driving frequency in the output setting information (at step S303). Further, the control unit 16 detects the driving current and the driving voltage set by the output control unit 14 based on the detected current and voltage signal S4 received from the output control unit 14, and stores the detected driving current and driving voltage in the storage unit 16a as the output setting information. Accordingly, the control unit 16 completes making a driving preparation for driving the ultrasonic vibrator 2a to output the ultrasonic vibration having the desired amplitude at the resonance frequency Fr to the probe 3, thus turning into the driving waiting state (at step S304).

If the abnormality determination unit 16 determines that the output frequency equal to the resonance frequency Fr is out of the frequency range, i.e., if the output control unit 14 cannot detect the resonance frequency Fr within the frequency range, the output frequency corresponding to the detected frequency signal S3 is not equal to the resonance frequency Fr within the frequency range. If so, the control unit 16 cannot detect the resonance frequency Fr within the frequency range ("No" at step S302). The abnormality determination unit 16b determines that the frequency abnormality occurs in the ultrasonic surgical system 10 (at step S305). Thus, the control unit 16 detects the frequency abnormality occurring in the ultrasonic surgical system 10.

Alternatively, the abnormality determination unit 16b may calculate an absolute value of the deviation between the output frequency detected by the control unit 16 and the nominal frequency in the first determination criterion information or the second determination criterion information, and determine whether the absolute value of the deviation exceeds the frequency deviation limit in the first determination criterion information or the second determination criterion information. In this alternative, the abnormality determination unit 16b determines that the frequency abnormality occurs in the ultrasonic surgical system 10 if the absolute value of the deviation exceeds the frequency deviation limit in the first determination criterion information or the second determination criterion information.

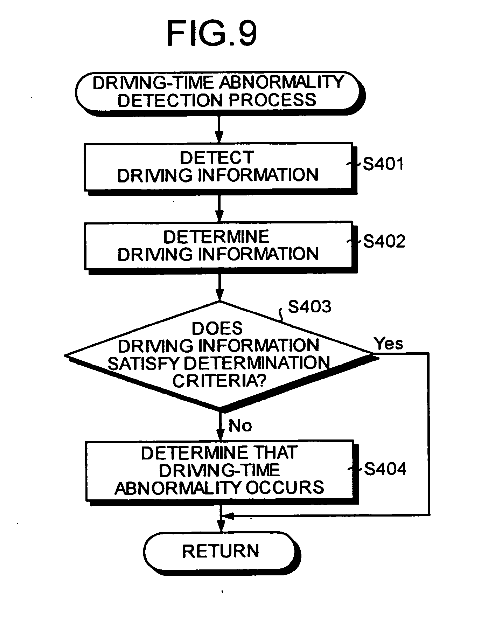

Fig. 9 is a flowchart for detailed explanation of respective processing steps until the control unit 16 detects the driving-time abnormality occurring in the ultrasonic surgical system 10 at step S106. Referring to Fig. 9, the control unit 16 exercises the vibrator driving control over the output control unit 14 if receiving the driving start instruction signal from the switch detection unit 11. In the vibrator driving control, the output control unit 14 controls the ultrasonic vibrator 2a to be driven under control of the control unit 16. The ultrasonic vibrator 2a outputs the ultrasonic vibration having the desired amplitude to the probe 3 under driving control of the output control unit 14. In addition, the output control unit 14 detects the output frequency (i.e., the resonance frequency Fr) of the driving signal S5 for supplying the electric energy to the ultrasonic vibrator 2a, the driving current, and the driving voltage. The output control unit 14 then transmits the detected frequency signal S3 corresponding to the output frequency and the detected current and voltage signal S4 corresponding to the driving current and the driving voltage to the control unit 16. The control unit 16 detects the output frequency, the driving current, and the driving voltage if the ultrasonic vibrator 2a is driven, based on the detected frequency signal S3 and the detected current and voltage signal S4 received from the output control unit 14. The information generation unit 16c generates the first accumulated time information corresponding to the accumulated time for which the ultrasonic vibration is output to the probe 3 and the second accumulated time information corresponding to the accumulated time for which the ultrasonic vibrator 2a outputs the ultrasonic vibration. The information generation unit 16c also calculates and outputs the capacitance of the ultrasonic vibrator 2a using the driving current and the driving voltage detected by the control unit 16. If so, the control unit 16 detects the output frequency, driving current, and driving voltage thus obtained, the capacitance calculated and output by the information generation unit 16c, the latest first accumulated time corresponding to the first accumulated time information generated by the information generation unit 16c and the latest second accumulated output time corresponding to the second accumulated time information generated by the information generation unit 16c as driving information on the ultrasonic vibrator 2b (at step S401), and stores the detected driving information in the storage unit 16a.

Thereafter, the abnormality determination unit 16b determines whether the pieces of driving information stored in the storage unit 16a satisfy respective determination criteria in the first determination criterion information or the second determination criterion information read from the storage units 2b and 3b in the information read processing, using the respective determination criteria (at step S402). For instance, the abnormality determination unit 16b determines whether the output frequency is within a frequency range set by the frequency upper limit and the frequency lower limit in the first determination criterion information or the second determination criterion information. The abnormality determination unit 16b determines whether the driving voltage exceeds the driving voltage upper limit in the first determination criterion information or the second determination criterion information. The abnormality determination unit 16b determines whether the driving current is within a current range set by the driving current upper limit and the lower limit driving current in the first determination criterion information or the second determination criterion information. In addition, the abnormality determination unit 16b determines whether the latest first accumulated output time exceeds the first output limit time in the first determination criterion information, and determines whether the latest second accumulated output time exceeds the second output limit time in the second determination criterion information. Further, the abnormality determination unit 16b determines whether the capacitance exceeds the capacitance limit in the second determination criterion information.

If the pieces of driving information do not satisfy the respective determination criteria for the driving information as a result of step S402 ("No" at step S403), the abnormality determination unit 16b determines that the driving-time abnormality occurs in the ultrasonic surgical system 10 (at step S404). For instance, if the output frequency is out of the frequency range set by the frequency upper limit and the frequency lower limit in the first determination criterion information or the second determination criterion information, the abnormality determination unit 16b determines that the frequency abnormality occurs in the ultrasonic surgical system 10. The control unit 16 detects the frequency abnormality occurring in the ultrasonic surgical system 10. If the driving voltage exceeds the driving voltage upper limit in the first determination criterion information or the second determination criterion information, the abnormality determination unit 16b determines that the driving voltage abnormality occurs in the ultrasonic surgical system 10. The control unit 16 detects the driving voltage abnormality occurring in the ultrasonic surgical system 10. If the driving current is out of the current range set by the driving current upper limit and the lower limit driving current in the first determination criterion information or the second determination criterion information, the abnormality determination unit 16b determines that the driving current abnormality occurs in the ultrasonic surgical system 10. The control unit 16 detects the driving current abnormality occurring in the ultrasonic surgical system 10. If the driving current is lower than the driving current upper limit, in particular, the abnormality determination unit 16b can determine that the driving current abnormality resulting from the overload on the handpiece 2 or the probe 3 occurs in the ultrasonic surgical system 10. In addition, if the driving current exceeds the driving current upper limit, the abnormality determination unit 16b can determine that the driving current abnormality resulting from the excessive output of the ultrasonic vibration occurs in the ultrasonic surgical system 10. Furthermore, if the latest first accumulated output time exceeds the first output limit time in the first determination criterion information, the abnormality determination unit 16b determines that the accumulated time abnormality of the probe 3 occurs in the ultrasonic surgical system 10. The control unit 16 detects the accumulated time abnormality of the probe 3 occurring in the ultrasonic surgical system 10. If the latest second accumulated output time exceeds the second output limit time in the second determination criterion information, the abnormality determination unit 16b determines that the accumulated time abnormality of the handpiece 2 occurs in the ultrasonic surgical system 10. The control unit 16 detects the accumulated time abnormality of the handpiece 2 occurring in the ultrasonic surgical system 10. If the capacitance exceeds the capacitance limit in the second determination criterion information, the abnormality determination unit 16b determines that the handpiece temperature abnormality occurs in the ultrasonic surgical system 10. The control unit 16 detects the handpiece temperature abnormality occurring in the ultrasonic surgical system 10.

The abnormality determination unit 16b can determine whether the probe temperature abnormality occurs in the ultrasonic surgical system 10 based on a frequency change speed by the time the output frequency changes to the frequency that is out of the frequency range. If so, the information generation unit 16c calculates and outputs this frequency change speed based on a change quantity of the output frequency sequentially detected as the driving information and the output time information received from the time calculation unit 15. If the frequency change speed obtained is lower than a predetermined frequency change speed limit, the abnormality determination unit 16 can determine that the probe temperature abnormality occurs in the ultrasonic surgical system 10. The control unit 16 detects the probe temperature abnormality occurring in the ultrasonic surgical system 10.

Alternatively, the abnormality determination unit 16b may calculate the absolute value of the deviation between the output frequency of the driving information and the nominal frequency in the first determination criterion information or the second determination criterion information. The abnormality determination unit 16 may also determine whether the absolute value of the deviation exceeds the frequency deviation limit in the first determination criterion information or the second determination criterion information. In this alternative, if the absolute value of the deviation exceeds the frequency deviation limit in the first determination criterion information or the second determination criterion information, the abnormality determination unit 16b can determine that the frequency abnormality occurs in the ultrasonic surgical system 10. The control unit 16 detects the frequency abnormality occurring in the ultrasonic surgical system 10. Further, the information generation unit 16c may calculate and output a speed (deviation change speed) at which the deviation between the output frequency and the nominal frequency changes, based on the output frequency and the nominal frequency sequentially detected as the driving information and the output time information received from the time calculation unit 15. In this alternative, if the deviation change speed thus obtained is lower than a predetermined deviation change speed limit, the abnormality determination unit 16b can determine that the probe temperature abnormality occurs in the ultrasonic surgical system 10. The control unit 16 detects the probe temperature abnormality occurring in the ultrasonic surgical system 10.

If the pieces of the driving information satisfy the respective determination criteria as a result of step S402 ("No" at step S403), the abnormality determination unit 16b determines that the driving-time abnormality does not occur in the ultrasonic surgical system 10. If so, the ultrasonic surgical system 10 is in a normal state in which no driving-time abnormality occurs in the system 10.

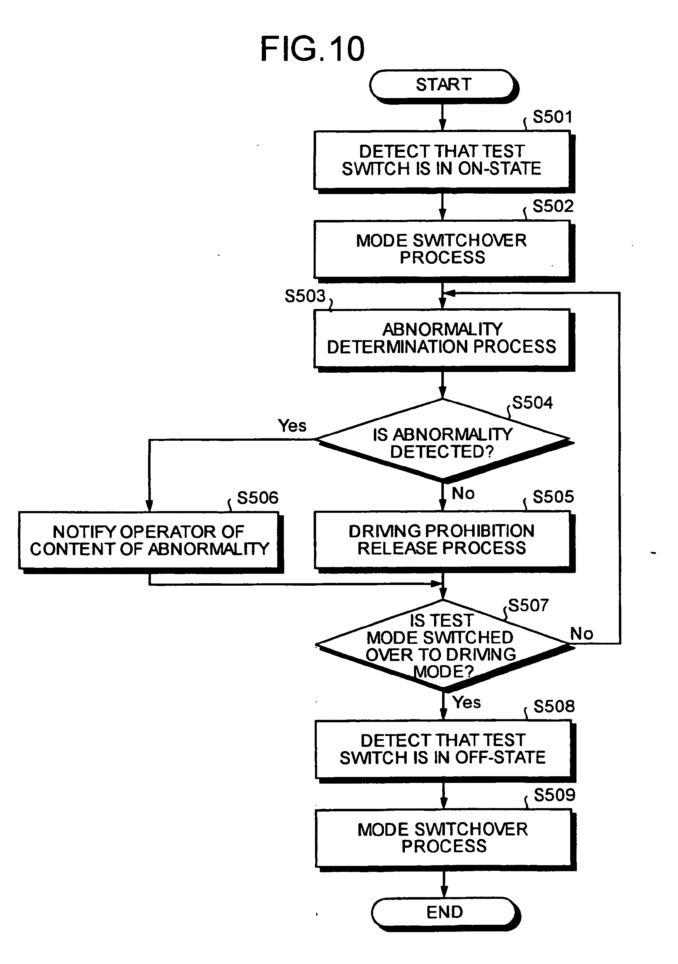

Respective processing steps until the control unit 16 permits the ultrasonic surgical system 10 the driving of which is stopped by the driving prohibition processing to be driven again will be explained in detail. Fig. 10 is a flowchart for detailed explanation of the respective processing steps until the control unit 16 permits the ultrasonic surgical system 10 the driving of which is stopped to be driven again, if the control unit 16 determines whether the abnormality occurs in the ultrasonic surgical system 10 the driving of which is stopped and detects the abnormality occurring in the ultrasonic surgical system 10. Referring to Fig. 10, if inputting the switch-on information from the test switch 1c, the switch detection unit 11 detects that the test switch 1c is in an ON-state (at step S501) and transmits the test mode switchover signal to the control unit 16. If receiving the test mode switchover signal from the switch detection unit 11, the control unit 16 performs a mode switchover processing for switching over a driving mode for exercising the output setting control or the vibrator driving control to a test mode for determining whether an abnormality occurs in the ultrasonic surgical system 10 (at step S502). If the control unit 16 is set in the test mode by this mode switchover processing, the control unit 16 releases the prohibition of the reception of the setting start instruction signal, thereby turning into a state in which the control unit 16 can exercise the output setting control.

Specifically, if receiving the setting start signal from the switch detection unit 11, then the control unit 16 exercises the output setting control over the output control unit 14, and the output control unit 14 sets the driving current and the driving voltage and performs the frequency sweep processing. If so, the output control unit 14 transmits the detected frequency signal S3 corresponding to the output frequency obtained by the frequency sweep processing and the detected current and voltage signal S4 corresponding to the set driving current and driving voltage to the control unit 16. The control unit 16 detects the output frequency based on the detected frequency signal S3 received from the output control unit 14, and detects the driving current and the driving voltage based on the detected current and voltage signal S4 received from the output control unit 14. The information generation unit 16c calculates and outputs the capacitance of the ultrasonic vibrator 2a using the driving current and the driving voltage detected by the control unit 16. The information generation unit 16c also calculates and outputs the frequency change speed or the deviation change speed using the output frequency detected by the control unit 16.

The abnormality determination unit 16b performs the abnormality determination processing by performing the same determination processing as that executed at step S402 using the output frequency, the driving current, the driving voltage, the capacitance, and the frequency change speed or the deviation change speed thus obtained. Specifically, the abnormality determination unit 16 determines whether the frequency abnormality, the driving current abnormality, the driving voltage abnormality, the handpiece temperature abnormality, or the probe temperature abnormality occurs in the ultrasonic surgical system 10 (at step S503).

The control unit 16 preferably exercises the output setting control over the output control unit 14 so that the output control unit 14 performs the frequency sweep processing using the rated current of the handpiece 2 or the probe 3 in the test mode. By doing so, it is possible to promote deteriorating the handpiece 2 or the probe 3 intentionally, and to facilitate determining whether an abnormality occurs in the ultrasonic surgical system 10. The control unit 16 further preferably controls the output control unit 14 so as to keep forcedly outputting the ultrasonic vibration from the ultrasonic vibrator 2a to the probe 3 for the predetermined time (e.g., about five seconds), if the output control unit 14 detects the resonance frequency and exercises the PLL control. This is based on the fact that an occurrence rate of mechanical destruction caused by the stress decreases exponentially with the passage of time of applying the stress. In addition, it is thereby possible to improve a reliability of the ultrasonic surgical system 10 for which it is determined in the abnormality determination processing at step S503 that no abnormality occurs in the system 10.

If the abnormality determination unit 16b determines that no frequency abnormality, no driving current abnormality, no driving voltage abnormality, no handpiece temperature abnormality, and no probe temperature abnormality occur in the ultrasonic surgical system 10 ("No" at step S504), then the control unit 16 performs a driving prohibition release processing to thereby release the prohibition of the reception of the instruction by the setting start instruction signal or the driving start instruction signal in the driving mode, and erases the abnormality history information stored in the storage units 2b and 3b (at step S505). Alternatively, the control unit 16 may transmit the instruction signal for outputting an output sound indicating that this driving prohibition release processing is accomplished to the sound source circuit 17a or the amplification circuit 17b, transmit the instruction signal for outputting an output display indicating that this driving prohibition release processing is accomplished to the display circuit 18, so as to notify the operator that no abnormality occurs in the ultrasonic surgical system 10 and that the driving prohibition release processing is accomplished.

If the abnormality determination unit 16 determines that the frequency abnormality, the driving current abnormality, the driving voltage abnormality, the handpiece temperature abnormality, or the probe temperature abnormality occurs in the ultrasonic surgical system 10, and the control unit 16 detects the abnormality determined by the abnormality determination unit 16b ("Yes" at step S504), the control unit 16 transmits the instruction signal for outputting the output sound corresponding to the detected abnormality to the sound source circuit 17a or the amplification circuit 17b, or transmits the instruction signal for outputting the output display corresponding to the detected abnormality to the display circuit 18, so as to notify the operator of occurrence of the abnormality to the ultrasonic surgical system 10 (at step S506).

Thereafter, if the test mode of the control unit 16 is continued ("No" at step S507), the respective processing steps after step S503 are repeatedly executed. If the test mode of the control unit 16 is switched over to the driving mode ("Yes" at step S507), the operator operates the test switch 1c and inputs the switch-off information on the test switch 1c to the switch detection unit 11. If inputting the switch-off information from the test switch 1c, the switch detection unit 11 detects that the test switch 1c is in an OFF state (at step S508), and transmits the driving mode switchover signal to the control unit 16. If receiving the driving mode switchover signal from the switch detection unit 11, the control unit 16 performs the mode switchover processing for switching over the test mode to the driving mode (at step S509). If performing the driving prohibition release processing, the control unit 16 releases the prohibition of the reception of the setting start instruction signal and the driving start instruction signal, thereby turning into a state in which the control unit 16 can exercises the output setting control or the vibrator driving control based on the received instruction signals. Accordingly, the control unit 16 releases the prohibition of the output of the ultrasonic vibration from the ultrasonic vibrator 2a, and permits the ultrasonic vibrator 2a to be driven again.

According to the first embodiment, the instance of applying the present invention to the scissors type ultrasonic surgical system for incising the treatment target such as the biological tissue has been explained as one example of the ultrasonic surgical system. However, the present invention is not limited to the embodiment. The present invention may be applied to a lithotrity type ultrasonic surgical system for fracturing and aspirating a calculus in a body cavity or the like, a hook type ultrasonic surgical system for carrying out peeling, incision, or the like to the biological tissue or the like, an aspiration type ultrasonic surgical system for emulsifying and aspirating the biological tissue or the like, and various other ultrasonic surgical systems such as an ultrasonic scalpel.

According to the first embodiment, the control unit 16 reads the vibrator output control information and the second determination criterion information stored in the storage unit 2b and the probe output control information and the first determination criterion information stored in the storage unit 3b by performing the information read processing. However, the present invention is not limited to the embodiment. The control unit 16 may read the second determination criterion information stored in the storage unit 2b and the first determination criterion information stored in the storage unit 3b by the information read processing, and may set the output setting information by operating the up switch 1e, the down switch 1f, or the like provided on the controller 1.

As explained so far, according to the first embodiment, storage units are arranged in the probe for carrying out the desired medical treatment to the treatment target using the ultrasonic vibration and the handpiece that includes the ultrasonic vibrator to which the probe is connected, respectively. The storage unit arranged in the probe stores the determination criterion information on abnormalities of the ultrasonic surgical system including those resulting from the probe. The storage unit arranged in the handpiece stores the determination criterion information on abnormalities of the ultrasonic surgical system including those resulting from the handpiece. If the ultrasonic surgical system is turned on, the information read processing for reading the determination criterion information from the storage unit arranged in the probe, and for reading the determination criterion information from the storage unit arranged in the handpiece is carried out. It is determined whether the determination criterion information can be normally read by the information read processing. It is determined whether the connection abnormality occurs in the ultrasonic surgical system. It is also determined whether the combination abnormality occurs in the ultrasonic surgical system based on the result of the comparison between the determination criterion information read from the storage unit of the probe and that read from the storage unit of the handpiece. In addition, if the driving of the ultrasonic vibrator is controlled and the ultrasonic vibration is output from the ultrasonic vibrator to the probe, then it is determined whether the driving-preparation-time abnormality or the driving-time abnormality occurs in the ultrasonic surgical system based on the determination criterion information read from the storage unit of the probe or that read from the storage unit of the handpiece, and based on various pieces of information on driving control over the ultrasonic vibrator such as the output frequency, the driving current, and the driving voltage. If the driving-preparation-time abnormality or the driving-time abnormality of the ultrasonic surgical system is detected, the driving of the ultrasonic vibrator is stopped and the output of the ultrasonic vibration from the ultrasonic vibrator is prohibited. Therefore, the abnormality resulting from the connection state between the ultrasonic vibrator and the probe or that between the handpiece and the controller, and the abnormality resulting from the combination between the probe and the ultrasonic vibrator can be detected before the ultrasonic vibrator outputs the ultrasonic vibration to the probe. If the ultrasonic vibrator outputs the ultrasonic vibration to the probe, then the abnormality occurring in the ultrasonic surgical system can be detected at an early timing and the output of the ultrasonic vibration by the ultrasonic surgical system for which the driving-preparation-time abnormality or the driving-time preparation is detected can be prohibited. The load on the probe or the handpiece can be thereby reduced, and the deterioration of the probe or the handpiece can be thereby suppressed. Thus, the ultrasonic surgical system having enhanced system safety and improved operation efficiency can be realized. By using this ultrasonic surgical system, the operator can carry out the medical treatment to the treatment target efficiently and safely.

Further, according to the first embodiment, if the driving-preparation-time abnormality or the driving-time preparation occurring in the ultrasonic surgical system is detected, the abnormality history information that is a history of occurrence of the detected driving-preparation-time abnormality or driving-time preparation is stored, as the determination criterion information on the abnormality, in the storage unit of the probe or that of the handpiece. Therefore, the abnormality history information on the driving-preparation-time abnormality or the driving-time preparation can be followed up for each probe or handpiece. Before the ultrasonic vibrator outputs the ultrasonic vibration to the probe, it is possible to detect that the probe or the handpiece having this abnormality history information is used by the ultrasonic surgical system. It is possible to ensure detecting the abnormality resulting from the probe or the abnormality resulting from the handpiece without accelerating the deterioration of the probe or the handpiece.

Furthermore, according to the first embodiment, the control unit included in the ultrasonic surgical system can switch over between the driving mode, in which the ultrasonic vibrator is controlled to be driven so as to carry out the medical treatment to the treatment target, and the test mode, in which it is determined whether the abnormality resulting from the probe or the handpiece occurs. In the test mode, if the control unit can determine that the abnormality does not occur in the ultrasonic surgical system, then the control unit erases the abnormality history information stored in at least one of the storage unit of the probe and that of the handpiece, and releases the prohibition of the output of the ultrasonic vibration by the driving prohibition processing. Therefore, it is possible to promptly and efficiently drive the ultrasonic vibrator, the driving of which is stopped by the driving prohibition processing, again if the abnormality occurring in the ultrasonic surgical system is eliminated.

Moreover, according to the first embodiment, the accumulated output time at which the ultrasonic vibrator outputs the ultrasonic vibration to the probe is calculated for each of the probe and the handpiece. The accumulated output time of the probe is stored in the storage unit of the probe as the abnormality determination criterion information. The accumulated output time of the handpiece is stored in the storage unit of the handpiece as the abnormality determination criterion information. Therefore, the accumulated output time can be followed up for each probe or each handpiece. Before the ultrasonic vibrator outputs the ultrasonic vibration to the probe, it is possible to detect the probe or the handpiece the accumulated output time of which exceeds the predetermined specified time. If the ultrasonic vibrator outputs the ultrasonic vibration to the probe, and the accumulated output time of the probe or that of the handpiece exceeds the predetermined specified time, then the driving prohibition processing is performed and the output of the ultrasonic vibration by the ultrasonic surgical system can be thereby prohibited. Thus, the ultrasonic surgical system capable of ensuring prohibiting the output of the ultrasonic vibration using the probe or the handpiece, the accumulated output time of which exceeds the predetermined specified time, without accelerating the deterioration of the probe or the handpiece, and having further enhanced safety can be realized.

A second embodiment of the present invention will be explained hereinafter. According to the first embodiment, the abnormality resulting from the deterioration, the damage, or the like of the probe or the handpiece is detected using the determination criterion information stored in the storage unit of the probe or that stored in the storage unit of the handpiece. According to the second embodiment, a short-circuit detection unit that detects electric conduction if the jaw is contacted with the probe is provided, and an abnormality resulting from deterioration of the jaw is detected.

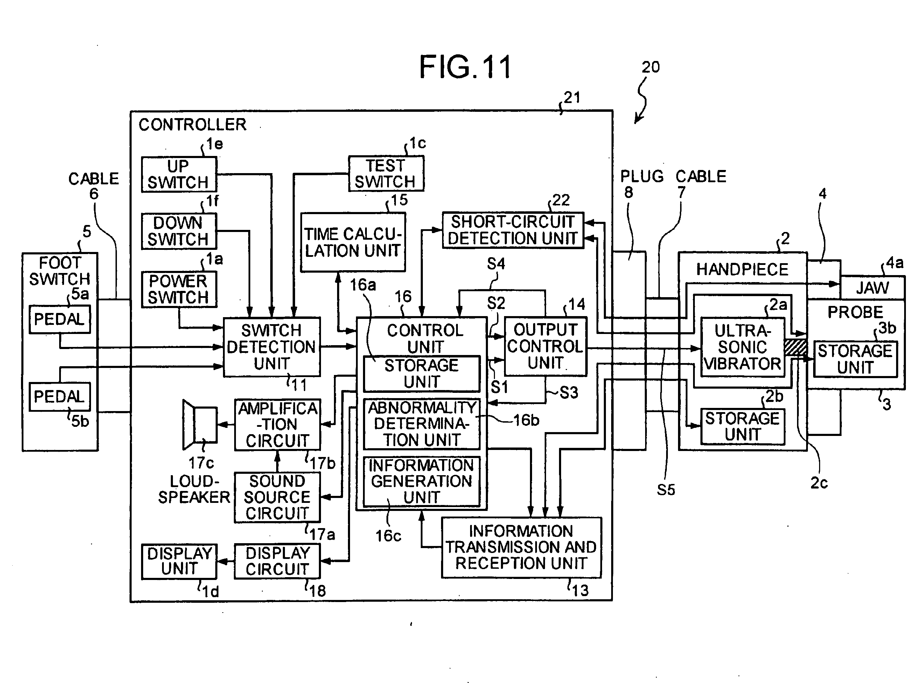

Fig. 11 is a block diagram of basic configuration of an ultrasonic surgical system according to the second embodiment of the present invention. A controller 21 in this ultrasonic surgical system 20 is the same as the controller 1 in the ultrasonic surgical system 10 according to the first embodiment except that a short-circuit detection unit 22 that detects the electric conduction between the jaw 4a of the sheath 4 and the probe 3 is provided in the controller 21. Since the other constituent elements according to the second embodiment are the same as those according to the first embodiment, the same constituent elements are denoted by the same reference symbols as those according to the first embodiment.

If the plug 8 of the handpiece 2 to which the probe 3 and the sheath 4 are connected is connected to the connector 1b of the controller 21, the short-circuit detection unit 22 is electrically connected to the probe 3 through the cable 7 and the handpiece 2, and electrically connected to the jaw 4a through the cable 7, the handpiece 2, and the sheath 4. If the probe 3, the jaw 4a, and the short-circuit detection unit 22 form a closed circuit, the short-circuit detection unit 22 functions to detect a continuity resistance of the closed circuit, and to perform a short-circuit detection processing for detecting the electric conduction between the probe 3 and the jaw 4a based on the continuity resistance.

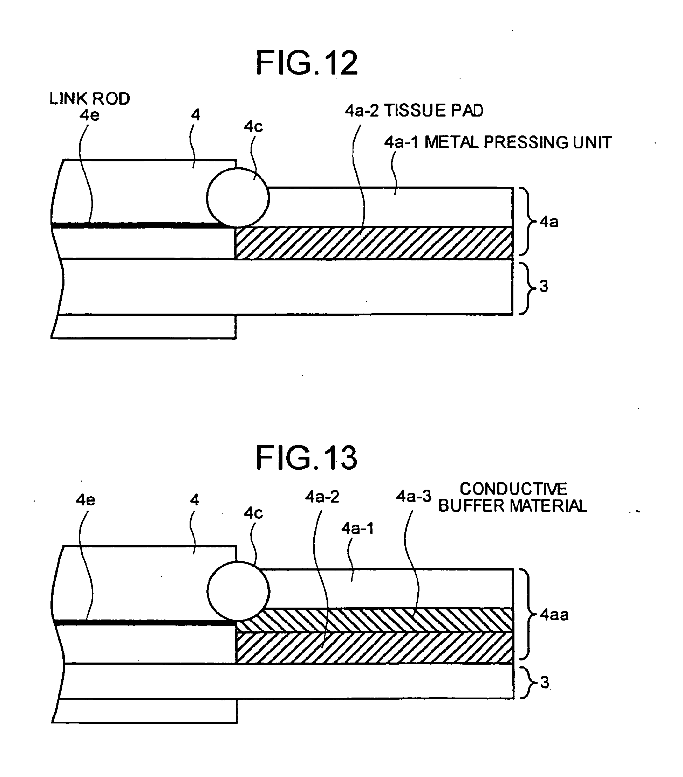

Fig. 12 is a typical view of an instance in which the jaw 4a is closed relative to the probe 3 by the operation of the grip 4b of the sheath 4 or the like. In Fig. 12, the jaw 4a includes a metal pressing unit 4a-1 made of a metal member such as stainless steel, and a tissue pad 4a-2 made of a resin member such as Teflon (trademark). The metal pressing unit 4a-1 includes a shaft 4c, and a link rod 4e operating longitudinally relative to a shaft direction of the sheath 4 while interlocking with the operation of the grip 4b is connected to the shaft 4c. The tissue pad 4a-2 is provided on a side of the metal pressing unit 4a-1 on which the unit 4a-1 contacts with the probe 3. The metal pressing unit 4a-1 performs an opening and closing operation relative to the probe 3 so as to press the tissue pad 4a-2 against the probe 3 about the shaft 4c by allowing the link rod 4e to operate longitudinally while interlocking with the operation of the grip 4b. The jaw 4a can thereby perform the opening and closing operation relative to the probe 3.

As explained above, the short-circuit detection unit 22 is electrically connected to the probe 3 through the cable 7 and the handpiece 2, and to the metal pressing unit 4a-1 of the jaw 4a through the cable 7, the handpiece 2, and the link rod 4e of the sheath 4. If the jaw 4a is closed relative to the probe 3, the probe 3, the metal pressing unit 4a-1 of the jaw 4a, and the short-circuit circuit 22 form a closed circuit through the tissue pad 4a-2. If so, the tissue pad 4a-2 functions as an electric resistance of this closed circuit.

If the ultrasonic vibration is output to the probe 3, then the tissue pad 4a-2 is worn by a friction between the tissue pad 4a-2 and the probe 3 or the treatment target. If the wearing progresses, the metal pressing unit 4a-1 is exposed to the treatment target or the probe 3. Following the progress of this wearing, the tissue pad 4a-2 deteriorates its function as the electric resistance of the closed circuit. Specifically, a continuity resistance of the closed circuit by the tissue pad 4a-2 is reduced if the wearing progresses. If the probe 3 contacts with the metal pressing unit 4a-1 exposed from the tissue pad 4a-2, the continuity resistance is rapidly reduced.

Alternatively, as a pressing unit that presses the treatment target against the probe 3, a jaw 4aa having a conductive buffer layer provided between the metal pressing unit 4a-1 and the tissue pad 4a-2 can be used in place of the jaw 4a. Fig. 13 is a typical view of an instance in which the jaw 4aa that is a modification of the jaw 4a is closed relative to the probe 3 by the operation of the grip 4b of the sheath 4 or the like. In Fig. 13, the jaw 4aa has a structure in which the conductive buffer material 4a-3 is provided between the metal pressing unit 4a-1 and the tissue pad 4a-2 of the jaw 4a.

The conductive buffer material 4a-3 is made of conductive resin such as silicon-based resin or carbon-based resin. The conductive buffer material 4a-3 functions as a conductor electrically connected to the metal pressing unit 4a-1, and also functions as a buffer that hampers contact between the probe 3 and the metal pressing unit 4a-1 and that relaxes an impact caused by the contact of the metal pressing unit 4a-1 with the probe 3. A conductive resistance of the conductive buffer 4a-3 can be set at a desired resistance by adjusting a content of a conductive material such as carbon contained in the buffer 4a-3.

The short-circuit unit 22 is electrically connected to the probe 3 similarly to the instance of using the jaw 4a, and electrically connected to the conductive buffer 4a-3 through the cable 7, the handpiece 2, the link rod 4e of the sheath 4, and the metal pressing unit 4a-1 of the jaw 4a. If the jaw 4a is closed relative to the probe 3, the probe 3, the conductive buffer 4a-2 of the jaw 4a, and the short-circuit detection unit 22 form a closed circuit through the tissue pad 4a-2. If so, the tissue pad 4a-2 functions as an electric resistance of the closed circuit.

If the ultrasonic vibration is output to the probe 3, then the tissue pad 4a-2 is worn by the friction between the tissue pad 4a-2 and the probe 3 or the treatment target. If the wearing progresses, the conductive buffer 4a-3 is exposed to the treatment target or the probe 3. The continuity resistance of the closed circuit by the tissue pad 4a-2 is reduced if the wearing progresses. If the probe 3 contacts with the conductive buffer 4a-3 exposed from the tissue pad 4a-2, the continuity resistance is rapidly reduced.

If the probe 3 is in contact with the conductive buffer 4a-3, the conductive buffer 4a-3 conducts an electric signal to the metal pressing unit 4a-1 or the probe 3, and relaxes an impact caused by the contact with the probe 3. However, the conductive buffer 4a-3 is worn by the friction with the probe 3 if relaxing the impact caused by the contact with the probe. If this wearing progresses to expose the metal pressing unit 4a-1, then the probe 3 contacts with the metal pressing unit 4a-1 and the conductive buffer 4a-3 loses its function as a buffer. Therefore, a thickness of the conductive buffer 4a-3 may be set equal to or larger than a thickness of the resin that is worn by the friction with the probe 3 since the probe 3 contacts with the conductive buffer 4a-3 until the output of the ultrasonic vibration to the probe 3 is prohibited. The thickness of the conductive buffer 4a-3 may be, for example, one millimeter. The conductive buffer 4a-3 can thereby ensure hampering the contact between the probe 3 and the metal pressing unit 4a-1, and preventing the jaw 4aa or the probe 3 from being damaged.

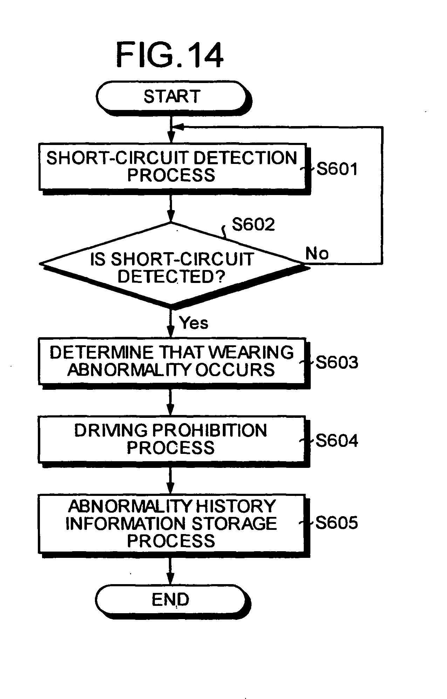

An operation of the control unit 16 for detecting a wearing abnormality resulting from the wearing of the jaw 4aa, and prohibiting the output of the ultrasonic vibration to the probe 3 will next be explained. The operation of the control unit 16 will be explained hereafter for the instance of using the jaw 4a. However, even if the jaw 4aa is used, the control unit 16 similarly operates. Fig. 14 is a flowchart of respective processing steps at which if the short-circuit detection unit 22 detects the electric conduction between the probe 3 and the jaw 4a, the control unit 16 detects the wearing abnormality and prohibits the output of the ultrasonic vibration to the probe 3. Referring to Fig. 14, if the controller 21 is turned on by the operation of the power switch 1a, the switch detection unit 11 detects that the power switch 1a is in an ON-state. Thereafter, the switch detection unit 11 transmits the information read instruction signal to the control unit 16, and transmits an instruction signal (a short-circuit detection instruction signal) for starting a short-circuit detection processing by the short-circuit detection unit 22 to the control unit 16. If receiving the information read instruction signal from the switch detection unit 11, the control unit 16 reads at least the determination criterion information from the storage units 2b and 3b as already explained. Specifically, the control unit 16 reads a continuity resistance limit serving as a criterion as to whether an electric signal is conducted between the probe 3 and the jaw 4a as the determination criterion information read from the storage units 2b and 3b. If receiving the short-circuit detection instruction signal from the switch detection unit 11, then the control unit 16 transmits conduction determination criterion information corresponding to the continuity resistance read from the storage units 2b and 3b to the short-circuit detection unit 22, and controls the short-circuit detection unit 22 to perform the short-circuit detection processing. The short-circuit detection unit 22 constantly transmits a feeble current signal to the probe 3 or the jaw 4a, constantly detects the continuity resistance of the closed circuit that conducts the electric signal, and detects the electric continuity between the probe 3 and the jaw 4a based on a result of comparison between the continuity resistance and the continuity determination criterion information received from the control unit 16 under control of the control unit 16 (at step S601). Specifically, the short-circuit detection unit 22 detects the continuity resistance of the closed circuit based on a current and a voltage of the electric signal, and compares the continuity resistance limit corresponding to the conduction determination criterion information with the detected continuity resistance. Further, if the continuity resistance is lower than the continuity resistance limit, the short-circuit detection unit 22 detects the electric conduction between the probe 3 and the jaw 4a, i.e., a short-circuit between the probe 3 and the jaw 4a resulting from the wearing of the tissue pad 4a-2. If the jaw 4a is closed relative to the probe 3, the control unit 16 may control the short-circuit detection unit 22 to detect the electric continuity between the probe 3 and the jaw 4a.

If detecting the short-circuit between the probe 3 and the jaw 4a ("Yes" at step S602), the short-circuit detection unit 22 transmits a short-circuit detection signal indicating that the unit 22 detects this short-circuit to the control unit 16. If the control unit 16 receives the short-circuit detection signal from the short-circuit detection unit 22, then the abnormality determination unit 16b determines that the wearing abnormality occurs in the ultrasonic surgical system 20 based on this short-circuit detection signal (at step S603). Thus, the control unit 16 detects the wearing abnormality occurring in the ultrasonic surgical system 20.

The "wearing abnormality" means herein an abnormality resulting from wearing of the tissue pad 4a-2 by the friction between the tissue pad 4a-2 and the probe 3 or the treatment target. Namely, the wearing abnormality is such that the metal pressing unit 4a-1 of the jaw 4a or the conductive buffer material 4a-3 of the jaw 4aa is exposed, thereby deteriorating efficiency of the medical treatment carried out to the treatment target and causing a damage of the probe 3.

If detecting this wearing abnormality, the control unit 16 prohibits the output of the ultrasonic vibration from the ultrasonic vibrator 2a, controls the output of the output sound or output display corresponding to the detected wearing abnormality, and notifies the operator of occurrence of the wearing abnormality to the ultrasonic surgical system 20 (at step S604). Thereafter, the information generation unit 16c generates the abnormality history information corresponding to the detected wearing abnormality, and the control unit 16 stores the abnormality history information generated by the information generation unit 16c in the storage units 2b and 3b (at step S605). Alternatively, the control unit 16 may perform the abnormality history information storage processing at step S605 either simultaneously with or before the driving prohibition processing at step S604. The control unit 16 may omit the abnormality history information storage processing at step S605. In this alternative, the information generation unit 16c does not generate the abnormality history information corresponding to the wearing abnormality, and the control unit 16 does not store the abnormality history information corresponding to the wearing abnormality in the storage units 2b and 3b.

After being notified of occurrence of the wearing abnormality at step S604, the operator can recognize that the wearing abnormality occurs in the ultrasonic surgical system 20, and replace the sheath 4 including the defective jaw 4a by another one without causing the damage of the probe 3, the malfunction of the handpiece 2, or the like. In addition, after completing replacement of the sheath 4, the operator can carry out the abnormality determination processing to the ultrasonic surgical system 20 by the operation of the test switch 1c or the like. If it is determined that no wearing abnormality occurs in the ultrasonic surgical system 20, the control unit 16 can permit the ultrasonic vibrator 2a the driving of which is stopped by the driving prohibition processing performed due to the wearing abnormality to be driven again.

According to the second embodiment, the short-circuit detection unit 22 detects the continuity resistance of the closed circuit formed by the probe 3, the jaw 4a, and the short-circuit detection unit 22. In addition, the short-circuit detection unit 22 detects the short-circuit between the probe 3 and the jaw 4a based on the result of the comparison between the continuity resistance and the continuity resistance limit serving as the continuity determination criterion. However, the present invention is not limited to this embodiment. The short-circuit detection unit 22 may detect the current of the electric signal conducted to the closed circuit formed by the probe 3, the jaw 4a, and the short-circuit detection unit 22, and may detect the short-circuit between the probe 3 and the jaw 4a based on the result of the comparison between the current and the current limit serving as the continuity determination criterion.

According to the second embodiment, the short-circuit detection unit 22 is provided in the controller 21 separately from the control unit 16. However, the present invention is not limited to this embodiment. The short-circuit detection unit 22 may be provided in the control unit 16.

As explained so far, according to the second embodiment, if the ultrasonic surgical system is turned on, the electric signal is constantly transmitted to the probe or the jaw. If the closed circuit including the probe and the jaw is formed, then the continuity resistance of the closed circuit is detected based on the current and the voltage of the electric signal conducted to this closed circuit, and the short-circuit between the probe and the jaw is detected based on the detected continuity resistance. Therefore, before the ultrasonic vibration is output, the contact between the jaw having the worn tissue pad and the probe can be detected. If the ultrasonic vibration is output, the contact between the jaw having the worn tissue pad and the probe can be detected at an early timing, and the output of the ultrasonic vibration can be prohibited. Thus, it is possible to realize the ultrasonic surgical system capable of reducing the load on the probe or the handpiece, suppressing the deterioration of the probe or the handpiece, replacing the jaw deteriorated by the wearing of the tissue pad or the like with another jaw efficiently, and carrying out the medical treatment to the treatment target efficiently and safely.

Further, by using the jaw having the conductive buffer layer provided between the tissue pad and the metal pressing unit, even if the tissue pad is worn, this conductive buffer relaxes the impact caused by the contact with the probe and realizes the electric continuity with the probe. Therefore, the contact between the probe and the metal pressing unit can be hampered, and the wearing of the tissue pad can be detected. It is thereby possible to prevent the damage of the probe during a surgical operation, improve operation efficiency, and further enhance operation safety.

## Claims

1. An ultrasonic surgical system (10; 20), comprising:
a handpiece (2) including an ultrasonic vibrator (2a), and a first storage unit (2b) that stores first determination criterion information being a criterion of whether an abnormality occurs in the ultrasonic surgical system;
a probe (3) adapted to be connected to the handpiece (2), the probe (3) including a second storage unit (3b) that stores second determination criterion information as a criterion of whether an abnormality occurs in the ultrasonic surgical system, wherein the probe (3) is to be connected to the ultrasonic vibrator (2a), and transmits ultrasonic vibrations output from the ultrasonic vibrator (2a) to a treatment target; and
a control unit (16) which determines whether the abnormality occurs in the ultrasonic surgical system based on the first determination criterion information and the second determination criterion information, and which stops driving the ultrasonic vibrator (2a) if determining that the abnormality occurs in the ultrasonic surgical system.

2. The ultrasonic surgical system (10; 20) according to claim 1, wherein
the control unit (16) stores as the first determination criterion information history information on abnormality that has occurred in the ultrasonic surgical system, in the first storage unit (2b).

3. The ultrasonic surgical system (10; 20) according to claim 2, further comprising:
a switchover unit (5) that switches over between a driving mode in which the control unit (16) controls the ultrasonic vibrator (2a) and a test mode in which whether an abnormality resulting from the handpiece (2) occurs in the ultrasonic surgical system is determined, wherein
in the test mode, if the abnormality resulting from the handpiece (2) is not determined, the control unit (16) erases the history information from the first storage unit (2b) and permits the ultrasonic vibrator (2a), driving of which is stopped, to be driven again.

4. The ultrasonic surgical system (10; 20) according to claim 1, further comprising a time calculation unit (15) that calculates an output time at which the ultrasonic vibrator (2a) outputs the ultrasonic vibration to the handpiece (2), wherein
the control unit (16) calculates an accumulated output time of the handpiece (2) based on the output time, and stores the accumulated output time as the first determination criterion information, in the first storage unit.

5. The ultrasonic surgical system (10; 20) according to claim 1, wherein
the control unit (16) stores as the second determination criterion information history information on abnormality that has occurred in the ultrasonic surgical system, in the second storage unit (2b).

6. The ultrasonic surgical system (10; 20) according to claim 5, further comprising:
a switchover unit (5) that switches over between a driving mode in which the control unit (16) controls the ultrasonic vibrator (2a) and a test mode in which whether an abnormality resulting from the probe (3) occurs in the ultrasonic surgical system is determined, wherein
in the test mode, if the abnormality resulting from the probe (3) is not determined, the control unit (16) erases the history information from the second storage unit (3b) and permits the ultrasonic vibrator (2a), driving of which is stopped, to be driven again.

7. The ultrasonic surgical system (10; 20) according to claim 1, further comprising a time calculation unit (15) that calculates an output time at which the ultrasonic vibrator (2a) outputs the ultrasonic vibration to the probe (3),
wherein
the control unit (16) calculates an accumulated output time of the probe (3) based on the output time, and stores the accumulated output time as the second determination criterion information, in the second storage unit (3b).

8. The ultrasonic surgical system (10; 20) according to claim 1, further comprising:
a pressing unit (4a) that presses the treatment target against the probe (3); and
a conduction detection unit (22) that detects electric conduction between the pressing unit (4a) and the probe (3) based on at least one of the first determination criterion information and the second determination criterion information when the pressing unit (4a) contacts with the probe, wherein
the control unit (16), when receiving detection information indicating detection of the electric conduction between the pressing unit (4a) and the probe (3) from the conduction detection unit (22), determines that the abnormality occurs in the ultrasonic surgical system, and stops driving the ultrasonic vibrator (2a).

9. A method of detecting an abnormality of an ultrasonic surgical system (10; 20) which includes: a handpiece (2) having an ultrasonic vibrator (2a) and a first storage unit (2b); and a probe (3) adapted to be connected to the handpiece (2), the probe (3) having a second storage unit (3b) and being connected to the ultrasonic vibrator (2a) to transmit ultrasonic vibrations output from the ultrasonic vibrator to a treatment target, the method comprising:
determining whether an abnormality occurs in the ultrasonic surgical system (10; 20) based on first determination criterion information being a criterion of whether an abnormality occurs in the ultrasonic surgical system stored in the first storage unit (2b) of the handpiece (2) and second determination criterion information as a criterion of whether an abnormality occurs in the ultrasonic surgical system stored in the second storage unit (3b) of the probe (3) before the ultrasonic vibrator (2a) is driven;
determining whether a resonance point of the ultrasonic vibrator (2a) is detected within a frequency range set by one of the first determination criterion information and the second determination criterion information if the abnormality is not determined before the ultrasonic vibrator (2a) is driven;
determining that an abnormality occurs in the ultrasonic surgical system (10; 20) if the resonance point is not detected within the frequency range;
determining whether an abnormality occurs to the ultrasonic surgical system (10; 20) based on driving information obtained during driving of the ultrasonic vibrator (2a) and based on one of the first determination criterion information and the second determination criterion information if the abnormality is not determined based on the resonance point; and
stopping driving the ultrasonic vibrator (2a) when the abnormality is determined in the ultrasonic surgical system.

10. The method according to claim 9, further comprising storing, in one of the storage units (2b, 3b), history information on the abnormality as one of the first determination criterion information and the second determination criterion information if the abnormality is determined.

11. The abnormality detection method according to claim 10, further comprising:
switching from a driving mode in which the ultrasonic vibrator (2a) is driven, to a test mode in which an abnormality of one of the probe (3) and the handpiece (2) is determined, after the driving of the ultrasonic vibrator (2a) is stopped and the history information is stored;
in the test mode, if the abnormality is not determined, erasing the history information from the storage unit (2b, 3b) and permitting the ultrasonic vibrator (2a), driving of which is stopped, to be driven again; and
switching from the test mode to the driving mode after the ultrasonic vibrator (2a) is permitted.

12. A computer program product for detecting an abnormality of an ultrasonic surgical system (10; 20) which includes a control unit; a handpiece (2) having an ultrasonic vibrator (2a) and a first storage unit (2b); and a probe (3) adapted to be connected to the handpiece (2), the probe (3) having a second storage unit (3b) and being connected to the ultrasonic vibrator (2a) to transmit ultrasonic vibrations output from the ultrasonic vibrator to a treatment target, including computer executable instructions stored on a computer readable medium, wherein the instructions, when executed by the control unit, cause the control unit to perform:
determining whether an abnormality occurs in the ultrasonic surgical system (10; 20) based on first determination criterion information being a criterion of whether an abnormality occurs in the ultrasonic surgical system stored on a first storage unit (2b) on the handpiece (2) and second determination criterion information as a criterion of whether an abnormality occurs in the ultrasonic surgical system stored on a second storage unit (3b) on the probe (3) before the ultrasonic vibrator (2a) is driven;
determining whether a resonance point of the ultrasonic vibrator (2a) is detected within a frequency range set by one of the first determination criterion information and the second determination criterion information if the abnormality is not determined before the ultrasonic vibrator (2a) is driven;
determining that an abnormality occurs in the ultrasonic surgical system (10; 20) if the resonance point is not detected within the frequency range;
determining whether an abnormality occurs to the ultrasonic surgical system (10; 20) based on driving information obtained during driving of the ultrasonic vibrator (2a) and based on one of the first determination criterion information and the second determination criterion information if the abnormality is not determined based on the resonance point; and
stopping driving the ultrasonic vibrator (2a) when the abnormality is determined in the ultrasonic surgical system.

13. The computer program product according to claim 12, wherein the instructions further cause the computer to perform storing, in one of the storage units (2b, 3b), history information on the abnormality as one of the first determination criterion information and the second determination criterion information if the abnormality is determined.

14. The computer program product according to claim 13, wherein the instructions further cause the computer to perform:
switching from a driving mode in which the ultrasonic vibrator (2a) is driven, to a test mode in which an abnormality of one of the probe (3) and the handpiece (2) is determined, after the driving of the ultrasonic vibrator (2a) is stopped and the history information is stored;
in the test mode, if the abnormality is not determined, erasing the history information from the storage unit (2b, 3b) and permitting the ultrasonic vibrator (2a), driving of which is stopped, to be driven again; and
switching from the test mode to the driving mode after the ultrasonic vibrator (2a) is permitted.

## Patentansprüche

1. Ein chirurgisches Ultraschallsystem (10; 20), das umfasst:
ein Handteil (2) mit einem Ultraschallvibrator (2a) und eine erste Speichereinheit (2), die ein erstes Informationsbestimmungskriterium speichert, das ein Kriterium dafür darstellt, ob eine abnormale Gerätsituation in dem chirurgischen Ultraschallsystem vorliegt,
eine Sonde (3), die sich mit dem Handteil (2) verbinden lässt, die eine zweite Speichereinheit (3b) aufweist, die ein zweites Informationsbestimmungskriterium als ein zweites Kriterium dafür speichert, ob eine abnormale Gerätesituation im Ultraschallsystem vorliegt,
bei dem die Sonde (3) mit dem Ultraschallvibrator (2) zu verbinden ist und die von dem Ultraschallvibrator (2) abgegebenen Ultraschallschwingungen an einen zu behandelnden Bereich überträgt,
und
eine Steuereinheit (16), die auf der Grundlage der ersten Informationsbestimmungskriteriums und des zweiten die Informationsbestimmungskriteriums feststellt, ob ein abnormaler Gerätezustand in dem Ultraschallsystem vorliegt, und die den Betrieb des Ultraschallvibrators (2) anhält, wenn festgestellt wird, dass eine abnormale Gerätesituation in dem chirurgischen Ultraschallsystem vorliegt.

2. Das chirurgische Ultraschallsystem (10; 20) nach Anspruch 1, bei dem
die Steuereinheit (16) als das Informationsbestimmungskriterium Informationen zum bisherigen Auftreten von abnormalen Gerätesituationen in dem Ultraschallsystem in der ersten Speichereinheit (2b) speichert.

3. Das chirurgische Ultraschallsystem (10; 20) nach Anspruch 2, das ferner umfasst:
eine Umschalteinheit (5), die zwischen einem Betriebsmodus, in dem die Steuereinheit (16) den Ultraschallvibrator (2a) steuert, und einem Prüfmodus, in dem geprüft wird, ob im Ultraschallsystem ein vom Handteil (2) herrührende abnormale Gerätesituation vorliegt, umschaltet, und bei dem
im Prüfmodus, wenn eine vom Handteil (2) herrührende abnormale Gerätesituation nicht festgestellt wird, die Steuereinheit (16) die Informationen über das bisherige Auftreten abnormaler Gerätesituation aus der ersten Speichereinheit (2b) löscht und es dem Ultraschallvibrator (2a), dessen Betrieb angehalten ist, ermöglicht, wieder betrieben zu werden.

4. Das chirurgische Ultraschallsystem (10; 20) nach Anspruch 1, das ferner
eine Zeitbrechnungseinheit (15) umfasst, die die Abgabezeitdauer, während der der Ultraschallvibrator (2a) Ultraschallschwingungen an das Handteil (2) ausgegeben hat, errechnet, und bei dem
die Steuereinheit (16) eine aufgelaufene Abgabezeitspanne des Handteils (2) auf der Grundlage der Abgabezeitdauer berechnet und die aufgelaufene Abgabezeitspanne als das erste Informationsbestimmungskriterium in der ersten Speichereinheit (2b) speichert.

5. Das chirurgische Ultraschallsystem (10; 20) nach Anspruch 1, bei dem
die Steuereinheit (16) als zweites Informationsbestimmungskriterium Informationen zum bisherigen Auftreten von abnormalen Gerätesituationen in dem chirurgischen Ultraschallsystem in der zweiten Speichereinheit (2b) speichert.

6. Das chirurgische Ultraschallsystem (10; 20) nach Anspruch 5, das ferner umfasst:
eine Umschalteinheit (5), die zwischen einem Betriebsmodus, in dem die Steuereinheit (16) den Ultraschallvibrator (2a) steuert, und einem Prüfmodus, in dem geprüft wird, ob im Ultraschallsystem ein von der Sonde (3) herrührender abnormaler Gerätezustand vorliegt, umschaltet, und bei dem
im Prüfmodus, wenn eine von der Sonde (3) herrührende abnormale Gerätesituation nicht festgestellt wird, die Steuereinheit (16) die Informationen über das bisherige Auftreten abnormaler Gerätesituationen aus der zweiten Speichereinheit (3b) löscht und es dem Ultraschallvibrator (2a), dessen Antrieb angehalten ist, ermöglicht, wieder betrieben zu werden.

7. Das chirurgische Ultraschallsystem (10; 20) nach Anspruch 1, die ferner
eine Zeitbrechnungseinheit (15) umfasst, die die Abgabezeitdauer, während der der Ultraschallvibrator (2a) Ultraschallschwingungen an die Sonde (3) abgibt, errechnet, und bei dem
die Steuereinheit (16) eine aufgelaufene Abgabezeitspanne der Sonde (3) auf der Grundlage der Abgabezeitdauer berechnet und die aufgelaufene Abgabezeitspanne als das zweite Informationsbestimmungskriterium in der zweiten Speichereinheit (3b) speichert.

8. Das chirurgische Ultraschallsystem (10; 20) nach Anspruch 1, das ferner umfasst:
eine Andrückeinheit (4a), die den zu behandelnden Bereich gegen die Sonde (3) andrückt, eine Leitungsmesseinheit (22), die elektrische Leitung zwischen der Andrückeinheit (4a) und der Sonde (3) auf der Grundlage des ersten Informationsbestimmungskriteriums und des zweiten Informationsbestimmungskriteriums bei Berührung der Sonde durch die Andrückeinheit (4a) misst,
und bei dem die Steuereinheit (16) beim Empfang von Messwerten, die die Messung einer elektrischen Leitung zwischen der Andrückeinheit (4a) und der Sonde (3) von der Leitungsmesseinheit (22) anzeigen, feststellt, dass eine abnormale Gerätesituation in dem chirurgischen Ultraschallsystem vorliegt und den Betrieb des Ultraschalvibrators (2a) anhält.

9. Ein Verfahren zum Erkennen einer abnormalen Gerätesituation in einem chirurgischen Ultraschallsystem (10; 20), das ein Handstück (2) mit einem Ultraschallvibrator (2a) und einer ersten Speichereinheit (2b) sowie eine Sonde (3), die sich mit dem Handteil (2) verbinden lässt, aufweist, wobei die Sonde (3) eine zweite Speichereinheit (3b) hat und mit dem Ultraschallvibrator (2a) verbunden wird, um von dem Ultraschallvibrator abgegebene Ultraschallschwingungen an einen zu behandelnden Bereich zu übertragen,
das umfasst:
das Feststellen, bevor der Ultraschallvibrator (2a) in Betrieb genommen wird, ob in dem chirurgischen Ultraschallsystem (10; 20) eine abnormale Gerätesituation vorliegt auf der Grundlage eines ersten Informationsbestimmungskriteriums, das ein Kriterium dafür darstellt, ob eine abnormale Gerätesituation in dem chirurgischen Ultraschallsystem vorliegt und in der ersten Speichereinheit (2b) des Handteils (2) gespeichert ist, und eines zweiten Informationsbestimmungskriteriums, das ein Kriterium dafür darstellt, ob ein abnormaler Gerätezustand in dem chirurgischen Ultraschallsystem vorliegt und in der zweiten Speichereinheit (3b) der Sonde (3) gespeichert ist,
das Bestimmen, ob ein Resonanzpunkt des Ultraschallvibrators (2a) innerhalb eines Frequenzbereichs festgestellt wird, der entweder von dem ersten Informationsbestimmungskriterium oder von dem zweiten Informationsbestimmungskriterium vorgegeben ist, wenn eine abnormale Gerätesituation vor der Inbetriebnahme des Ultraschallvibrator (2a) nicht festgestellt worden ist;
das Feststellen, dass eine abnormale Gerätesituation in dem chirurgischen Ultraschallsystem (10; 20) vorliegt, wenn der Resonanzpunkt nicht innerhalb des Frequenzbereichs festgestellt wird;
das Bestimmen, ob eine abnormale Gerätesituation in dem chirurgischen Ultraschallsystem (10; 20) vorliegt, auf der Grundlage von Betriebsinformationen, die während des Betriebs des Ultraschallvibrators (2a) gewonnen wurden, und auf der Grundlage entweder des ersten Informationsbestimmungskriteriums oder des zwei Informationsbestimmungskriteriums, wenn keine abnormale Gerätesituation auf der Grundlage des Resonanzpunkts bestimmt worden sind, und
das Anhalten des Betriebs des Ultraschallvibrators ist (2), wenn die abnormale Gerätesituation in dem chirurgischen Ultraschallsystem festgestellt worden ist.

10. Das Verfahren nach Anspruch 9, das ferner das Speichern von Informationen über das bisherige Auftreten von abnormalen Gerätesituationen entweder als erstes Informationsbestimmungskriterium oder als zweites Informationsbestimmungskriterium in einer der beiden Speichereinheiten (2b, 3b) umfasst, wenn die abnormale Gerätesituation festgestellt worden ist.

11. Das Verfahren zum Erkennen einer abnormalen Gerätesituation nach Anspruch 10, das ferner umfasst:
das Umschalten von einem Betriebsmodus, in dem der Ultraschallvibrator (2a) betrieben wird, zu einem Prüfmodus, in dem eine abnormale Gerätesituation entweder in der Sonde (3) oder in dem Handteil (2) bestimmt wird, nachdem der Ultraschallvibrator (2a) angehalten worden ist und die Informationen über den bisherigen Verlauf abnormaler Gerätesituationen gespeichert wurden;.
im Prüfmodus das Löschen des bisherigen Auftretens abnormaler Gerätesituationen aus den Speichereinheiten (2b, 3b), und es dem Ultraschallvibrator (2a), dessen Antrieb angehalten ist, wieder zu ermöglichen, betrieben zu werden, wenn eine abnormale Gerätesituation nicht festgestellt worden ist, und
das Umschalten aus dem Prüfmodus in den Betriebsmodus, nachdem dem Ultraschallvibrator (2a) freigegeben worden ist.

12. Ein Computerprogrammprodukt zum Erkennen einer abnormalen Gerätesituation in einem chirurgischen Ultraschallsystem (10; 20), das eine Steuereinheit, ein Handteil (2) mit einem Ultraschallvibrator (2a) und einer ersten Speichereinheit (2b) sowie eine Sonde (3), die sich mit dem Handteil (2) verbinden lässt, aufweist, wobei die Sonde (3) eine zweite Speichereinheit (3b) hat und sich mit dem Ultraschallvibrator (2a) verbinden läßt, um von dem Ultraschallvibrator abgegebene Ultraschallschwingungen an einen zu behandelnden Bereich zu übertragen, wobei die Sonde (3) eine zweite Speichereinheit (3b) aufweist und mit dem Ultraschallvibrator (2a) zur Übertragung von von dem Ultraschallvibrator abgegebenen Ultraschallschwingungen an einen zu behandelnden Bereich verbunden wird, mit von einem Computer ausführbaren Befehlen, die auf einem computerlesbaren Medium gespeichert sind, und bei dem die Befehle, wenn sie durch die Steuereinheit ausgeführt werden, bewirken, dass die Steuereinheit ausführt:
das Feststellen, bevor der Ultraschallvibrator (2a) in Betrieb genommen wird, ob in dem chirurgischen Ultraschallsystem (10; 20) eine abnormale Gerätesituation vorliegt, auf der Grundlage eines ersten Informationsbestimmungskriteriums, das ein Kriterium dafür darstellt, ob eine abnormale Gerätesituation in dem chirurgischen Ultraschallsystem vorliegt und
das in der ersten Speichereinheit (2b) des Handteils (2) gespeichert ist, und eines zweiten Informationsbestimmungskriteriums, das ein Kriterium dafür darstellt, ob eine abnormale Gerätesituation in dem chirurgischen Ultraschallsystem vorliegt und in der zweiten Speichereinheit (3b) der Sonde (3) gespeichert ist,
das Bestimmen, bevor der Ultraschallvibrator (2a) in Betrieb genommen wird, ob ein Resonanzpunkt des Ultraschallvibrators (2a) innerhalb eines Frequenzbereichs festgestellt wird, der entweder von dem ersten Informationsbestimmungskriterium oder von dem zweiten Informationsbestimmungskriterium vorgegeben ist, wenn eine abnormale Gerätesituation nicht festgestellt worden ist;
das Bestimmen, dass eine abnormale Gerätesituation in dem chirurgischen Ultraschallsystem (10; 20) vorliegt, wenn der Resonanzpunkt nicht innerhalb des Frequenzbereichs festgestellt wird;
das Bestimmen, ob eine abnormale Gerätesituation in dem chirurgischen Ultraschallsystem (10; 20) vorliegt, auf der Grundlage von Betriebsinformationen, die während des Betriebs des Ultraschallvibrators (2a) gewonnen wurden, und auf der Grundlage entweder des ersten Informationsbestimmungskriteriums oder des zwei Informationsbestimmungskriteriums, wenn keine abnormale Gerätesituation auf der Grundlage des Resonanzpunkts bestimmt worden ist, und
das Anhalten des Betriebs des Ultraschallvibrators (2), wenn die abnormale Gerätesituation in dem chirurgischen Ultraschallsystem festgestellt worden ist.

13. Das Computerprogrammprodukt nach Anspruch 12, bei dem die Befehle ferner den Computer veranlassen, eine Speicherung in einer der Speichereinheiten (2b, 3b) von Informationen über das bisherige Auftreten abnormaler Gerätesituationen als ein erstes Informationsbestimmungskriterium oder als ein zweites Informationsbestimmungskriterium, wenn die abnormale Gerätesituation festgestellt worden ist, durchzuführen.

14. Das Computerprogrammprodukt nach Anspruch 13, bei dem die Befehle ferner den Computer veranlassen auszuführen:
das Umschalten aus einem Betriebsmodus, in dem der Ultraschallvibrator (2a) betrieben wird, in einen Prüfmodus, in dem eine abnormale Gerätesituation in der Sonde (3) oder des Handteils (2) festgestellt wird, nachdem der Betrieb des Ultraschallvibrators (2a) angehalten wurde und die Informationen über abnormale Gerätesituationen gespeichert worden sind; in den Prüfmodus, wenn eine abnormale Gerätesituation nicht festgestellt worden ist,
das Löschen der Informationen über das Auftreten abnormaler Gerätesituationen aus der Speichereinheit (2b, 3b) und es dem Ultraschallvibrator (2a), dessen Betrieb angehalten ist, zu ermöglichen, wieder betrieben zu werden, und
das Umschalten aus dem Betriebsmodus in den Prüfmodus, nachdem der Ultraschallvibrator (2a) freigegeben worden ist.

## Revendications

1. Système chirurgical ultrasonore (10 ; 20), comprenant :
une pièce à main (2) incluant un vibrateur ultrasonore (2a), et une première unité de stockage (2b) qui stocke des premières informations de critère de détermination consistant en un critère destiné à déterminer si une anomalie se produit au sein du système chirurgical ultrasonore ;
une sonde (3) adaptée pour être connectée à la pièce à main (2), la sonde (3) incluant une deuxième unité de stockage (3b) qui stocke des deuxièmes informations de critère de détermination en tant que critère destiné à déterminer si une anomalie se produit au sein du système chirurgical ultrasonore, dans lequel la sonde (3) est à connecter au vibrateur ultrasonore (2a) et transmet des vibrations ultrasonores délivrées en sortie du vibrateur ultrasonore (2a) vers une cible de traitement ; et
une unité de commande (16) qui détermine si l'anomalie se produit au sein du système chirurgical ultrasonore sur la base des premières informations de critère de détermination et des deuxièmes informations de critère de détermination, et qui arrête d'entraîner le vibrateur ultrasonore (2a) si elle détermine que l'anomalie se produit au sein du système chirurgical ultrasonore.

2. Système chirurgical ultrasonore (10 ; 20) selon la revendication 1, dans lequel
l'unité de commande (16) stocke en tant que premières informations de critère de détermination des informations historiques sur une anomalie qui s'est produite au sein du système chirurgical ultrasonore, dans la première unité de stockage (2b).

3. Système chirurgical ultrasonore (10 ; 20) selon la revendication 2, comprenant en outre :
une unité de basculement (5) qui bascule entre un mode d'entraînement en lequel l'unité de commande (16) commande le vibrateur ultrasonore (2a), et un mode de test en lequel est déterminé si une anomalie résultant de la pièce à main (2) se produit au sein du système chirurgical ultrasonore, dans lequel
en mode de test, si l'anomalie résultant de la pièce à main (2) n'est pas déterminée, l'unité de commande (16) efface les informations historiques de la première unité de stockage (2b) et permet au vibrateur ultrasonore (2a), dont l'entraînement est arrêté, d'être entraîné de nouveau.

4. Système chirurgical ultrasonore (10 ; 20) selon la revendication 1, comprenant en outre une unité de calcul de temps (15) qui calcule un temps de sortie auquel le vibrateur ultrasonore (2a) délivre en sortie la vibration ultrasonore vers la pièce à main (2), dans lequel
l'unité de commande (16) calcule un temps de sortie cumulé de la pièce à main (2) sur la base du temps de sortie, et stocke le temps de sortie cumulé en tant que premières informations de critère de détermination, dans la première unité de stockage.

5. Système chirurgical ultrasonore (10 ; 20) selon la revendication 1, dans lequel
l'unité de commande (16) stocke en tant que deuxièmes informations de critère de détermination des informations historiques sur une anomalie qui s'est produite au sein du système chirurgical ultrasonore, dans la deuxième unité de stockage (2b).

6. Système chirurgical ultrasonore (10 ; 20) selon la revendication 5, comprenant en outre :
une unité de basculement (5) qui bascule entre un mode d'entraînement en lequel l'unité de commande (16) commande le vibrateur ultrasonore (2a), et un mode de test en lequel est déterminé si une anomalie résultant de la sonde (3) se produit au sein du système chirurgical ultrasonore, dans lequel
en mode de test, si l'anomalie résultant de la sonde (3) n'est pas déterminée, l'unité de commande (16) efface les informations historiques de la deuxième unité de stockage (3b) et permet au vibrateur ultrasonore (2a), dont l'entraînement est arrêté, d'être entraîné de nouveau.

7. Système chirurgical ultrasonore (10 ; 20) selon la revendication 1, comprenant en outre une unité de calcul de temps (15) qui calcule un temps de sortie auquel le vibrateur ultrasonore (2a) délivre en sortie la vibration ultrasonore vers la sonde (3), dans lequel
l'unité de commande (16) calcule un temps de sortie cumulé de la sonde (3) sur la base du temps de sortie, et stocke le temps de sortie cumulé en tant que deuxièmes informations de critère de détermination, dans la deuxième unité de stockage (3b).

8. Système chirurgical ultrasonore (10 ; 20) selon la revendication 1, comprenant en outre :
une unité de compression (4a) qui comprime la cible de traitement contre la sonde (3) ; et
une unité de détection de conduction (22) qui détecte une conduction électrique entre l'unité de compression (4a) et la sonde (3) sur la base d'au moins les unes entre les premières informations de critère de détermination et les deuxièmes informations de critère de détermination quand l'unité de compression (4a) est en contact avec la sonde, dans lequel
l'unité de commande (16), quand elle reçoit des informations de détection indiquant la détection de la conduction électrique entre l'unité de compression (4a) et la sonde (3) en provenance de l'unité de détection de conduction (22), détermine que l'anomalie se produit au sein du système chirurgical ultrasonore, et arrête l'entraînement du vibrateur ultrasonore (2a).

9. Procédé de détection d'une anomalie d'un système chirurgical ultrasonore (10 ; 20) qui inclut : une pièce à main (2) comportant un vibrateur ultrasonore (2a) et une première unité de stockage (2b) ; et une sonde (3) adaptée pour être connectée à la pièce à main (2), la sonde (3) comportant une deuxième unité de stockage (3b) et étant connectée au vibrateur ultrasonore (2a) pour transmettre des vibrations ultrasonores délivrées en sortie du vibrateur ultrasonore vers une cible de traitement, le procédé comprenant :
de déterminer si une anomalie se produit au sein du système chirurgical ultrasonore (10 ; 20) sur la base des premières informations de critère de détermination consistant en un critère destiné à déterminer si une anomalie se produit au sein du système chirurgical ultrasonore stockées dans la première unité de stockage (2b) de la pièce à main (2), et des deuxièmes informations de critère de détermination en tant que critère destiné à déterminer si une anomalie se produit au sein du système chirurgical ultrasonore stockées dans la deuxième unité de stockage (3b) de la sonde (3) avant que le vibrateur ultrasonore (2a) ne soit entraîné ;
de déterminer si un point de résonance du vibrateur ultrasonore (2a) est détecté à l'intérieur d'une plage de fréquence définie par les unes entre les premières informations de critère de détermination et les deuxièmes informations de critère de détermination si l'anomalie n'est pas déterminée avant que le vibrateur ultrasonore (2a) ne soit entraîné ;
de déterminer qu'une anomalie se produit au sein du système chirurgical ultrasonore (10 ; 20) si le point de résonance n'est pas détecté à l'intérieur de la plage de fréquence ;
de déterminer si une anomalie se produit pour le système chirurgical ultrasonore (10 ; 20) sur la base d'informations d'entraînement obtenues au cours de l'entraînement du vibrateur ultrasonore (2a) et sur la base des unes entre les premières informations de critère de détermination et les deuxièmes informations de critère de détermination si l'anomalie n'est pas déterminée sur la base du point de résonance ; et
d'arrêter d'entraîner le vibrateur ultrasonore (2a) quand l'anomalie est déterminée au sein du système chirurgical ultrasonore.

10. Procédé selon la revendication 9, comprenant en outre de stocker, dans l'une des unités de stockage (2b, 3b), des informations historiques sur l'anomalie en tant que les unes entre les premières informations de critère de détermination et les deuxièmes informations de critère de détermination si l'anomalie est déterminée.

11. Procédé selon la revendication 9, comprenant en outre :
de basculer d'un mode d'entraînement en lequel le vibrateur ultrasonore (2a) est entraîné, vers un mode de test en lequel une anomalie de l'une entre la sonde (3) et la pièce à main (2) est déterminée, après que l'entraînement du vibrateur ultrasonore (2a) ait été arrêté et que les informations historiques aient été stockées ;
en mode de test, si l'anomalie n'est pas déterminée, d'effacer les informations historiques de l'unité de stockage (2b, 3b) et de permettre au vibrateur ultrasonore (2a), dont l'entraînement est arrêté, d'être entraîné de nouveau ; et
de basculer du mode de test vers le mode d'entraînement après que l'entraînement du vibrateur ultrasonore (2a) ait été permis.

12. Produit de programme d'ordinateur destiné à détecter une anomalie d'un système chirurgical ultrasonore (10 ; 20) qui inclut ; une unité de commande, une pièce à main (2) comportant un vibrateur ultrasonore (2a) et une première unité de stockage (2b) ; et une sonde (3) adaptée pour être connectée à la pièce à main (2), la sonde (3) comportant une deuxième unité de stockage (3b) et étant connectée au vibrateur ultrasonore (2a) pour transmettre des vibrations ultrasonores délivrées en sortie du vibrateur ultrasonore vers une cible de traitement, incluant des instructions exécutables par ordinateur stockées sur un support lisible par ordinateur, dans lequel les instructions, quand elles sont exécutées par l'unité de commande, conduit l'unité de commande à réaliser les opérations consistant à :
déterminer si une anomalie se produit au sein du système chirurgical ultrasonore (10 ; 20) sur la base des premières informations de critère de détermination consistant en un critère destiné à déterminer si une anomalie se produit au sein du système chirurgical ultrasonore stockées dans la première unité de stockage (2b) de la pièce à main (2) et des deuxièmes informations de critère de détermination en tant que critère destiné à déterminer si une anomalie se produit au sein du système chirurgical ultrasonore stockées dans une deuxième unité de stockage (3b) de la sonde (3) avant que le vibrateur ultrasonore (2a) ne soit entraîné ;
de déterminer si un point de résonance du vibrateur ultrasonore (2a) est détecté à l'intérieur d'une plage de fréquence définie par les unes entre les premières informations de critère de détermination et les deuxièmes informations de critère de détermination si l'anomalie n'est pas déterminée avant que le vibrateur ultrasonore (2a) ne soit entraîné ;
de déterminer qu'une anomalie se produit au sein du système chirurgical ultrasonore (10 ; 20) si le point de résonance n'est pas détecté à l'intérieur de la plage de fréquence ;
de déterminer si une anomalie se produit pour le système chirurgical ultrasonore (10 ; 20) sur la base d'informations d'entraînement obtenues au cours de l'entraînement du vibrateur ultrasonore (2a) et sur la base des unes entre les premières informations de critère de détermination et les deuxièmes informations de critère de détermination si l'anomalie n'est pas déterminée sur la base du point de résonance ; et
d'arrêter d'entraîner le vibrateur ultrasonore (2a) quand l'anomalie est déterminée au sein du système chirurgical ultrasonore.

13. Produit de programme d'ordinateur selon la revendication 12, dans lequel les instructions conduisent en outre l'ordinateur à réaliser un stockage, dans l'une des unités de stockage (2b, 3b), d'informations historiques sur l'anomalie en tant que les unes entre les premières informations de critère de détermination et les deuxièmes informations de critère de détermination si l'anomalie est déterminée.

14. Produit de programme d'ordinateur selon la revendication 13, dans lequel les instructions conduisent en outre l'ordinateur à réaliser les opérations consistant à :
basculer d'un mode d'entraînement en lequel le vibrateur ultrasonore (2a) est entraîné, vers un mode de test en lequel une anomalie de l'une entre la sonde (3) et la pièce à main (2) est déterminée, après que l'entraînement du vibrateur ultrasonore (2a) ait été arrêté et que les informations historiques aient été stockées ;
en mode de test, si l'anomalie n'est pas déterminée, effacer les informations historiques de l'unité de stockage (2b, 3b) et permettre au vibrateur ultrasonore (2a), dont l'entraînement est arrêté, d'être entraîné de nouveau ; et
basculer du mode de test vers le mode d'entraînement après que l'entraînement du vibrateur ultrasonore (2a) ait été permis.
